# EUROPEAN PATENT APPLICATION

(11) **EP 4 652 977 A1**
(43) Date of publication of application: **26.11.2025**
(21) Application number: 24836193.3
(22) Date of filing: 23.05.2024
(51) Int. Cl.: A61H 1/02, B25J 9/00, B25J 9/16

(54) **WEARABLE DEVICE AND OPERATION METHOD THEREOF**

(30) Priority: 03.07.2023 KR 20230085809
(71) Applicant: Samsung Electronics Co., Ltd., Suwon-si, Gyeonggi-do 16677 (KR)
(72) Inventor: JU, Indon, Suwon-si Gyeonggi-do 16677 (KR); LEE, Byunghwa, Suwon-si Gyeonggi-do 16677 (KR)
(74) Representative: Gulde & Partner
(86) International application number: PCT/KR2024/006996
(87) International publication number: WO 2025/009744

(57) **Abstract**

This wearable device may comprise: a communication module for communicating with an electronic device worn on the body of a user; a driving module for generating torque and providing the generated torque to the user; at least one IMU sensor for acquiring movement information, which includes acceleration information and/or rotation angle information about the user; and a processor (processors). The processor(s) can: control the driving module so as to provide the torque to the user; receive, from the electronic device, through the communication module, sensing data acquired by the electronic device; determine the coordinate values of a first point corresponding to a first part of the body by using the received sensing data; receive the acquired movement information from the IMU; determine, on the basis of the received movement information, the coordinate values of each of second and third points corresponding to each of second and third parts of the body; and determine the pose of the user on the basis of the coordinate values of each of the first, second and third points.

## Description

### BACKGROUND

### 1. Field

Certain example embodiments relate to a wearable device and/or an operating method thereof.

### 2. Description of Related Art

In general, a walking assistance device may refer to a mechanism or device that helps a person exercise and/or that helps a patient who cannot walk on his own due to various diseases, accidents, and the like, to perform walking exercises for rehabilitation treatment. With the recent intensifying aging societies, a growing number of people experience inconvenience in walking or have difficulty in normal walking due to malfunctioning joint issues, and there is increasing interest in walking assistance devices. A walking assistance device is to be worn on a body of a user to assist the user with exercise and/or walking, such as by providing a desired or necessary muscular strength and to induce the user to walk in a normal walking pattern.

### SUMMARY

According to an example embodiment, a wearable device may include a communication module configured to communicate with an electronic device worn on a body of a user, a driving module, comprising a motor and/or circuitry, configured to generate a torque and provide the user with the generated torque, at least one inertial measurement unit (IMU) sensor configured to obtain motion information including at least one of acceleration information or rotation angle information of the user, and at least one processor comprising processing circuitry. The processor(s) may control the driving module to provide the torque to the user. The processor(s) may receive sensing data obtained by the electronic device from the electronic device through the communication module. The processor(s) may determine a coordinate value of a first point corresponding to a first part of the body using the received sensing data. The processor(s) may receive the obtained motion information from the IMU, and determine respective coordinate values of a second point and a third point respectively corresponding to a second part and a third part of the body based on the received motion information. The processor(s) may determine a posture of the user based on the respective coordinate values of the first point, the second point, and the third point.

According to an example embodiment, a wearable device may include a communication module (comprising communication circuitry) configured to communicate with an electronic device worn on a first part of a body of a user, a driving module configured to generate a torque and provide the user with the generated torque, a first IMU sensor configured to obtain first motion information including at least one of acceleration information or rotation angle information of a second part of the body, a second IMU sensor configured to obtain second motion information including at least one of acceleration information or rotation angle information of a second joint of a third part of the body, and at least one processor comprising processing circuitry. The processor(s) may control the driving module to provide the torque to the user. The processor(s) may receive sensing data obtained by the electronic device from the electronic device through the communication module. The processor(s) may determine a coordinate value of a first point corresponding to the first part using the received sensing data. The processor(s) may receive the first motion information and the second motion information respectively from the first IMU sensor and the second IMU sensor. The processor(s) may determine a coordinate value of a second point corresponding to the second part based on the received first motion information. The processor(s) may determine a coordinate value of a third point corresponding to the third part based on the received second motion information. The processor(s) may determine a posture of the user based on the respective coordinate values of the first point, the second point, and the third point.

According to an example embodiment, an operating method of a wearable device may include receiving sensing data obtained by an electronic device worn on a body of a user from the electronic device, determining a coordinate value of a first point corresponding to a first part of the body using the received sensing data, receiving motion information including at least one of acceleration information or rotation angle information of the user from an IMU of the wearable device, determining respective coordinate values of a second point and a third point respectively corresponding to a second part and a third part of the body based on the received motion information, and determining a posture of the user based on the respective coordinate values of the first point, the second point, and the third point.

According to an example embodiment, a wearable device may link with another wearable device (e.g., smart glasses or wireless earphones) to allow a user to monitor a real-time gait and posture (e.g., head posture or gait posture) of the user.

According to an example embodiment, a wearable device may link with another wearable device (e.g., smart glasses or wireless earphones) to provide a user with a recommended posture (e.g., recommended head posture or recommended gait posture), thereby allowing the user to correct his or her posture.

According to an example embodiment, a wearable device may perform a physical method (e.g., rotating or adjusting a driving module), thereby helping a user in correcting a posture.

### BRIEF DESCRIPTION OF THE DRAWINGS

The above and other aspects, features, and advantages of certain embodiments of the present disclosure will be more apparent from the following detailed description, taken in conjunction with the accompanying drawings, in which:
FIG. 1A is a diagram illustrating an overview of a wearable device worn on a body of a user according to an example embodiment;
FIG. 1B is a diagram illustrating an example of a system including a wearable device according to an example embodiment;
FIG. 2A is a rear schematic view of a wearable device according to an example embodiment;
FIG. 2B is a left side view of a wearable device according to an example embodiment;
FIGS. 3A to 3C are block diagrams illustrating examples of a configuration of a wearable device according to an example embodiment;
FIG. 4 is a diagram illustrating an interaction between a wearable device and an electronic device according to an example embodiment;
FIG. 5 is a diagram illustrating an example of a configuration of an electronic device according to an example embodiment;
FIGS. 6A and 6B are diagrams illustrating an example of determining a posture of a standing user by a wearable device according to an example embodiment;
FIGS. 7A and 7B are diagrams illustrating another example of determining a posture of a standing user by a wearable device according to an example embodiment;
FIG. 8 is a diagram illustrating an example of setting a first point of a user by a wearable device according to an example embodiment;
FIGS. 9A and 9B are diagrams illustrating examples of a calibration operation of a wearable device according to an example embodiment;
FIG. 10 is a diagram illustrating an example of determining a posture of a moving user by a wearable device according to an example embodiment;
FIG. 11 is a diagram illustrating an example of determining a posture of a user by a wearable device according to an example embodiment;
FIG. 12 is a diagram illustrating an example of determining a gait posture of a user by a wearable device according to an example embodiment;
FIGS. 13 and 14 are diagrams illustrating examples of screens in which a posture of a user is displayed according to an example embodiment;
FIGS. 15 and 16 are diagrams illustrating examples of operations for correcting a posture of a user by a wearable device according to an example embodiment;
FIGS. 17A and 17B are diagrams illustrating an example of an upper body-type wearable device according to an example embodiment;
FIG. 18 is a block diagram illustrating an example of a configuration of a control system of an upper body-type wearable device according to an example embodiment;
FIG. 19 is a diagram illustrating an example of determining a posture of a user by an upper body-type wearable device according to an example embodiment;
FIG. 20 is a diagram illustrating an example of an operation of a wearable device according to an example embodiment; and
FIG. 21 is a flowchart illustrating an example of an operating method of a wearable device according to an example embodiment.

### DETAILED DESCRIPTION

The following detailed structural or functional description is provided as an example only and various alterations and modifications may be made to the embodiments. Here, the embodiments are not construed as limited to the disclosure and should be understood to include all changes, equivalents, and replacements within the idea and the technical scope of the disclosure.

Terms, such as first, second, and the like, may be used herein to describe components. Each of these terminologies is not used to define an essence, order or sequence of a corresponding component but used merely to distinguish the corresponding component from other component(s). For example, a first component may be referred to as a second component, and similarly the second component may also be referred to as the first component.

It should be noted that if it is described that one component is "connected", "coupled", or "joined" to another component, at least a third component(s) may be "connected", "coupled", and "joined" between the first and second components, although the first component may be directly connected, coupled, or joined to the second component.

As used herein, the singular forms "a", "an", and "the" include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, embodiments will be described in detail with reference to the accompanying drawings. When describing the embodiments with reference to the accompanying drawings, like reference numerals refer to like elements and any repeated description related thereto will be omitted.

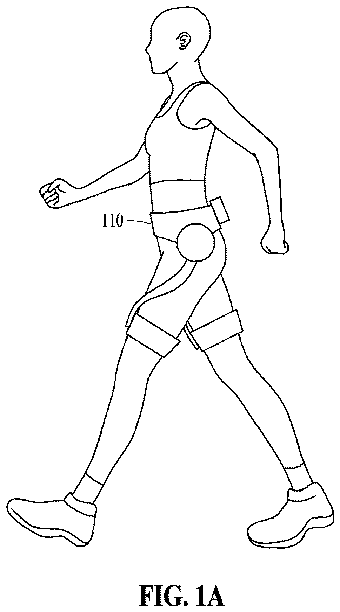

FIG. 1A is a diagram illustrating an overview of a wearable device worn on a body of a user according to an example embodiment.

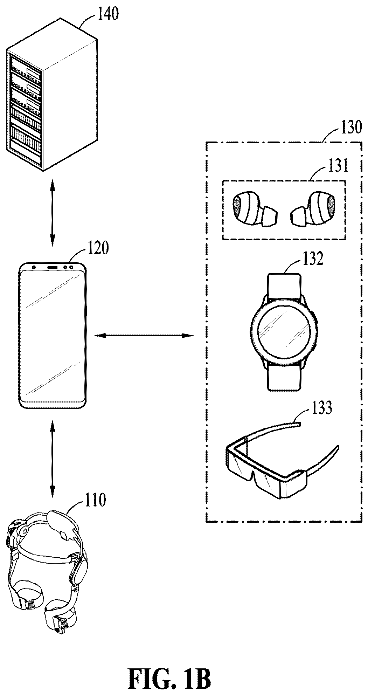

Referring to FIG. 1, a wearable device 110 may be a device worn on a body of a user to assist the user in walking, exercising, and/or working. In embodiments, the term "wearable device" may be replaced with "wearable robot," "walking assistance device," or "exercise assistance device". The user may be a human or an animal, but is not limited thereto. The wearable device 110 may be worn on a body (e.g., a lower body (the legs, ankles, knees, etc.), an upper body (the torso, arms, wrists, etc.), or the waist) of the user to provide an external force such as an assistance force and/or a resistance force to a body motion of the user. The assistance force may be a force applied in the same direction as the body motion direction of the user, and the resistance force may be a force applied in a direction opposite to the body motion direction of the user. The term "resistance force" may also be referred to as "exercise load".

When the wearable device 110 performs a walking assist function to assist the user in walking, the wearable device 110 may assist a portion or entirety of a leg of the user by providing an assistance force to the body of the user, thereby assisting the user in walking. The wearable device 110 may enable the user to walk independently or to walk for a long time by providing a force required for the user to walk, thereby extending the walking ability of the user. The wearable device 110 may help in improving an abnormal walking habit or gait posture of a walker.

When the wearable device 110 performs an exercise function to enhance the exercise effect of the user, the wearable device 110 may hinder a body motion of the user or provide resistance to a body motion of the user by providing a resistance force to the body of the user. When the wearable device 110 is, for example, a hip-type wearable device, the wearable device 110 may provide an exercise load to a body motion of the user while being worn on the legs, thereby enhancing the exercise effect of the user. The user may perform a walking motion while wearing the wearable device 110 for exercise. In this case, the wearable device 110 may apply a resistance force to the leg motion during the walking motion of the user.

In various example embodiments, an example of a hip-type wearable device 110 that is worn on the waist and legs is described for ease of description. However, as described above, the wearable device 110 may be worn on another body part (e.g., the upper arms, lower arms, hands, calves, and feet) other than the waist and legs (particularly, the thighs), and the shape and configuration of the wearable device 110 may vary depending on the body part on which the wearable device 110 is worn.

FIG. 1B is a diagram illustrating an example of a system including a wearable device according to an example embodiment.

Referring to FIG. 1B, a smart phone 120 may communicate with the wearable device 110 and remotely control the wearable device 110. The smart phone 120 may be various types of devices. The smart phone 120 may include, for example, a portable communication device, a computer device, a portable multimedia device, or a home appliance, but is not limited thereto. The smart phone 120 may be referred to as an electronic device.

According to an embodiment, the smart phone 120 and/or the wearable device 110 may be connected, directly or indirectly, to another wearable device 130. For example, the wearable device 110, the smart phone 120, and the other wearable device 130 may be connected to each other through a wireless communication link (e.g., a Bluetooth communication link). The other wearable device 130 may include, for example, wireless earphones 131, a smart watch 132, or smart glasses 133, but is not limited thereto. The wireless earphones 131 and the smart glasses 133 may correspond to wearable devices worn on the head. The other wearable device 130 may also be referred to as an accessory device. The smart watch 132 may be a watch-type wearable device (or a watch-type electronic device), and the smart glasses 133 may be an eyewear-type wearable device (or an eyewear-type electronic device). The smart glasses 133 may display, for example, augmented reality (AR) images.

In an embodiment, the smart watch 132 may control the wearable device 110. If the smart watch 132 is connected to the smart phone 120 via a wireless communication link, and the smart phone 120 is connected to the wearable device 110 via a wireless communication link, the smart watch 132 may control the wearable device 110 through the smart phone 120. Embodiments are not limited thereto, and the smart watch 132 may be directly connected to the wearable device 110 and control the wearable device 110.

In an embodiment, the smart phone 120 may transmit, to the other wearable device 130, a control signal to instruct to provide a user with feedback corresponding to a state of the wearable device 110. The other wearable device 130 may provide (or output) feedback (e.g., at least one of visual feedback, auditory feedback, or haptic feedback) corresponding to the state of the wearable device 110 in response to the reception of the control signal.

In an embodiment, the smart phone 120 may communicate with a server 140 using short-range wireless communication (e.g., Wi-Fi) or mobile communication (e.g., 4G, 5G, etc.).

In an embodiment, the smart phone 120 may receive profile information of the user from the user. The profile information may include, for example, at least one of the age, gender, height, weight, or body mass index (BMI), or a combination thereof. The smart phone 120 may transmit the profile information of the user to the server 140.

In an embodiment, the smart phone 120 and/or the wearable device 110 may request the user to perform one or more target motions to determine (or check) the exercise ability of the user. The one or more target motions may include, for example, a knee lift, a backward leg stretch, etc. The knee lift may be an exercise (or a motion) that starts from a position of the user standing straight with two feet on the ground and returns to the standing position after lifting a knee as much as possible without bending at the waist. The backward leg stretch may be an exercise (or a motion) that starts from a position of the user standing straight with the hands on the wall and returns to the standing position after lifting a leg backward as much as possible without bending at the waist.

In an embodiment, the wearable device 110 may obtain motion information of the user performing a target motion using a sensor (e.g., an inertial measurement unit (IMU)), and transmit the obtained motion information to the smart phone 120. The smart phone 120 may transmit the obtained motion information to the server 140.

In an embodiment, the server 140 may determine a target amount of exercise of the user for each of the exercise types (e.g., strength training, balance exercise, and aerobic exercise) through the profile information and motion information received from the smart phone 120. The server 140 may transmit the target amount of exercise for each exercise type to the smart phone 120.

In an embodiment, the server 140 may include a database in which information about a plurality of exercise programs to be provided to the user through the wearable device 110 is stored. For example, the server 140 may manage a user account of the user of the smart phone 120 or the wearable device 110. The server 140 may store and manage a workout program performed by the user and a result of performance with respect to the workout program in link with the user account.

In an embodiment, the smart phone 120 and/or the server 140 may provide the user with various exercise programs to achieve an exercise goal in various exercise environments desired by the user. The exercise goal may include, for example, at least one of muscle strength improvement, physical strength improvement, cardiovascular endurance improvement, core stability improvement, flexibility improvement, or symmetry improvement, or a combination thereof.

In an embodiment, the smart phone 120 and/or the server 140 may recommend exercise programs to the user to achieve the exercise goal of the user. Each exercise program may include one or more exercise modes. For example, each exercise mode may be about a body motion to achieve a predetermined exercise goal. For example, running may be an exercise mode for improving the cardiovascular endurance of the user. For example, a lunge may be an exercise mode for improving the core stability of the user. A combination of a plurality of exercise modes forming each exercise program may vary according to the exercise goal of the user. The smart phone 120 may provide the user with various exercise programs according to the combination of the plurality of exercise modes, even for the same exercise goal.

In an embodiment, the plurality of exercise modes may be stored in the smart phone 120 or the server 140 as a database. The smart phone 120 or the server 140 may generate the plurality of exercise programs based on a variety of information about the user and recommend a target exercise program among the plurality of exercise programs to the user in consideration of the exercise goal or an exercise performance state of the user. For example, the smart phone 120 or the server 140 may determine the target exercise program to recommend to the user based on at least one of the exercise goal, an exercise history, or an exercise performance result of the user. Accordingly, a new exercise program may be recommended to the user even if the user performs an exercise every day under the same exercise goal, and the user may feel like performing a different exercise from the previous exercise by performing the new exercise program.

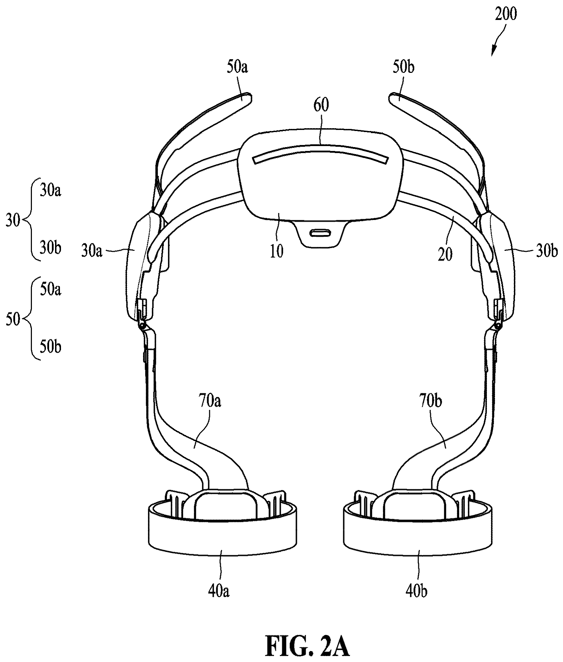

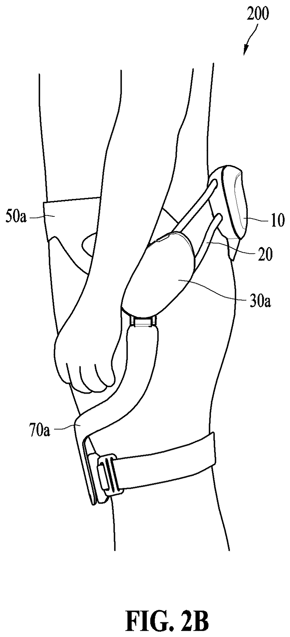

FIG. 2A is a rear schematic view of a wearable device according to an example embodiment. FIG. 2B is a left side view of a wearable device according to an example embodiment.

A wearable device 200 shown in FIGS. 2A and 2B may be an example of the wearable device 110.

Referring to FIG. 2A, according to an embodiment, the wearable device 200 may include a base body 10, a base frame 20, a driving module 30, fastening portions 40a and 40b, a main belt 50, and leg driving frames 70a and 70b. Each "driving module" herein may comprise one or more of a motor, a sensor(s), and/or circuitry.

According to an embodiment, the base body 10 may be positioned on the lumbar region (an area of the lower back) of the user while the user is wearing the wearable device 200. The base body 10 may be mounted on the lumbar region of the user to provide a cushioning feeling to the lower back of the user and may support the lower back of the user. The base body 10 may be hung on the hip region (an area of the hips) of the user to prevent or reduce chances of the wearable device 200 from being separated downward due to gravity while the user is wearing the wearable device 200. The base body 10 may distribute a portion of the weight of the wearable device 200 to the lower back of the user while the user is wearing the wearable device 200. The base body 10 may be connected, directly or indirectly, to the base frame 20. Base frame connecting elements (not shown) to be connected, directly or indirectly, to the base frame 20 may be provided at both end portions of the base body 10.

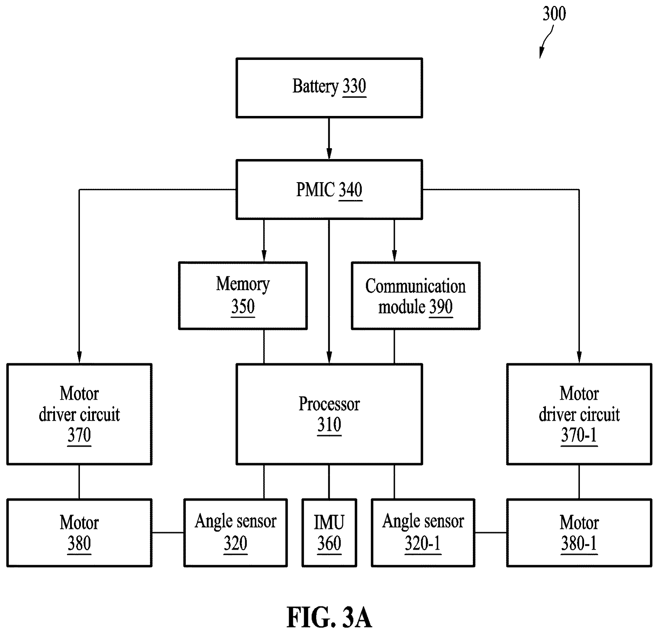

According to an embodiment, the base body 10 may include a lighting unit 60. The lighting unit 60 may include a plurality of light sources (e.g., light-emitting diodes (LEDs)). The lighting unit 60 may emit light by control of a processor (e.g., a processor 310 of FIGS. 3A to 3C). According to embodiments, the processor may control the lighting unit 60 such that visual feedback corresponding to the state of the wearable device 200 (e.g., a booting state, a sensing state, etc.) may be provided (or output) to the user through the lighting unit 60.

According to an embodiment, the base frame 20 may extend from both end portions of the base body 10. The lumbar region of the user may be accommodated inside the base frame 20. The base frame 20 may include at least one rigid body beam. Each beam may be in a curved shape having a preset curvature to enclose the lumbar region of the user. The main belt 50 may be connected, directly or indirectly, to an end portion of the base frame 20. The driving module 30 may be mounted on the base frame 20. The base frame 20 may include a connector (not shown) for mounting the driving module 30 thereon.

According to an embodiment, the driving module 30 may include a first driving module 30a positioned on the left side of the user while the user is wearing the wearable device 200, and a second driving module 30b positioned on the right side of the user while the user is wearing the wearable device 200.

According to an embodiment, the first driving module 30a may include a first angle sensor (e.g., a first encoder or a first Hall sensor) for measuring the left hip joint angle of the user. The second driving module 30b may include a second angle sensor (e.g., a second encoder or a second Hall sensor) for measuring the right hip joint angle of the user.

According to an embodiment, the first driving module 30a may include a first actuator and a first reducer, and the second driving module 30b may include a second actuator and a second reducer. An output end of the first actuator may be connected, directly or indirectly, to an input end of the first reducer, and an output end of the second actuator may be connected, directly or indirectly, to an input end of the second reducer.

According to an embodiment, the processor (e.g., the processor 310 described later) may determine a torque value (e.g., a torque value *τₗ*(*t*) to be described later) and control the first actuator to generate a torque based on the determined torque value (e.g., *τₗ*(*t*)). With this control, the first actuator may generate the torque, and the generated torque may be reduced by the first reducer. The torque reduced by the first reducer may rotate the first leg driving frame 70a. The torque reduced by the first reducer may be provided, for example, to the left leg of the user through the first leg driving frame 70a. The processor (e.g., the processor 310 described later) may determine a torque value (e.g., a torque value *τᵣ*(*t*) to be described later) and control the second actuator to generate a torque based on the determined torque value (e.g., *τᵣ*(*t*)). With this control, the second actuator may generate the torque, and the generated torque may be reduced by the second reducer. The torque reduced by the second reducer may rotate the second leg driving frame 70b. The torque reduced by the second reducer may be provided, for example, to the right leg of the user through the second leg driving frame 70b.

Each "processor" herein includes processing circuitry, and/or may include multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

According to an embodiment, the leg driving frames 70a and 70b may support the legs (e.g., thighs) of the user when the wearable device 200 is worn on the legs of the user. The leg driving frames 70a and 70b may include the first leg driving frame 70a configured to support the left leg of the user and the second leg driving frame 70b configured to support the right leg of the user.

According to an embodiment, the leg driving frames 70a and 70b may, for example, transmit the torques generated by the driving modules 30a and 30b (e.g., the torques reduced by the reducers) to the thighs of the user. As one end portions of the leg driving frames 70a and 70b are connected, directly or indirectly, to the driving modules 30a and 30b to rotate, and the other end portions of the leg driving frames 70a and 70b are connected, directly or indirectly, to the fastening portions 40a and 40b, the leg driving frames 70a and 70b may transmit the torques generated by the driving modules 30a and 30b to the thighs of the user while supporting the thighs of the user. For example, the leg driving frames 70a and 70b may push or pull the thighs of the user. The leg driving frames 70a and 70b may extend in the longitudinal direction of the thighs of the user. The leg driving frames 70a and 70b may be bent to surround at least a portion of the circumferences of the thighs of the user.

According to an embodiment, the fastening portions 40a and 40b may be connected, directly or indirectly, to the leg driving frames 70a and 70b and may fasten the leg driving frames 70a and 70b to the thighs. The fastening portions 40a and 40b may include a first fastening portion 40a configured to fasten the first leg driving frame 70a to the left thigh of the user and a second fastening portion 40b configured to fasten the second leg driving frame 70b to the right thigh of the user.

According to an embodiment, the first fastening portion 40a may include a first cover, a first fastening frame, and a first strap, and the second fastening portion 40b may include a second cover, a second fastening frame, and a second strap. The first cover and the second cover may be arranged on one sides of the thighs of the user or arranged around the knees (e.g., to be close to the knees between the thighs and knees). The first cover and the second cover may be arranged, for example, on the front surfaces of the thighs (or around the knees) of the user. The first cover and the second cover may be arranged in the circumferential directions of the thighs (or around the knees) of the user. The first cover and the second cover may extend to both sides from the other end portions of the leg driving frames 70a and 70b and may include curved surfaces corresponding to the thighs (or the knee areas) of the user. One ends of the first cover and the second cover may be connected to the fastening frames, and the other ends thereof may be connected to the straps.

According to an embodiment, the first fastening frame and the second fastening frame may be arranged, for example, to surround at least some portions of the circumferences of the thighs (or the knee areas) of the user, thereby preventing or reducing chances of the thighs of the user from being separated from the leg driving frames 70a and 70b. The first fastening frame may have a fastening structure that connects the first cover and the first strap, and the second fastening frame may have a fastening structure that connects the second cover and the second strap.

According to an embodiment, the first strap may enclose the remaining portion of the circumference of the left thigh (or the left knee area) of the user that is not covered by the first cover and the first fastening frame, and the second strap may enclose the remaining portion of the circumference of the right thigh (or the right knee area) of the user that is not covered by the second cover and the second fastening frame. The first strap and the second strap may include, for example, an elastic material (e.g., a band).

According to an embodiment, the main belt 50 may be connected to the base frame 20. The main belt 50 may include a first main belt 50a configured to enclose the left abdomen of the user while the user is wearing the wearable device 200, and a second main belt 50b configured to enclose the right abdomen of the user while the user is wearing the wearable device 200. The first main belt 50a may be formed in a shape having a longer length than the second main belt 50b, but is not limited thereto, and the first main belt 50a may be formed in a shape having the same length as or a shorter length than the second main belt 50b. The first main belt 50a and the second main belt 50b may be connected to both end portions of the base frame 20, respectively. The main belt 50 may be bent in a direction to surround the abdomen of the user when the body of the user is inserted in such a direction that it is accommodated in the wearable device 200. The first main belt 50a and the second main belt 50b may be connected to each other while the user is wearing the wearable device 200. The main belt 50 may distribute a portion of the weight of the wearable device 200 to the abdomen of the user while the user is wearing the wearable device 200.

Referring to FIG. 2B, the base body 10 may be mounted on the back of the lumbar region of the user and be hung on the hip region of the user, thereby supporting a portion of the weight of the wearable device 200. The first driving module 30a may be arranged on the left lumbar region of the user. The base frame 20 may extend from the end portion of the base body 10 and be inclined in a direction toward the first driving module 30a. The first main belt 50a mounted on the base frame 20 may surround the left abdomen of the user.

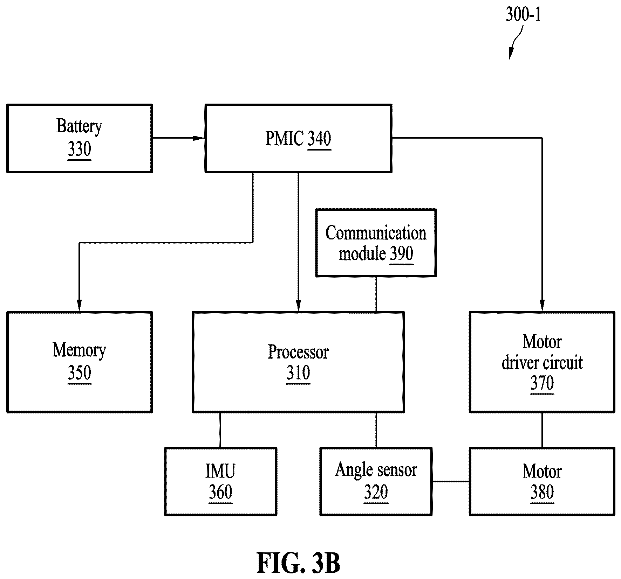

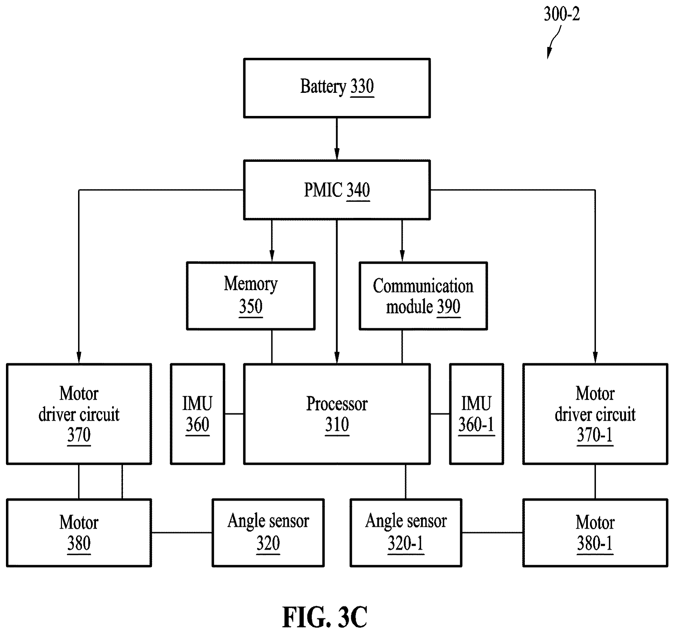

According to an embodiment, the wearable device 200 may include one or more IMUs (or IMU sensors). For example, one IMU (or an IMU sensor) (e.g., the IMU 360 of FIGS. 3A and 3B) may be positioned in the base body 10. As another example, the wearable device 200 may include the IMU 360-1 of FIG. 3C and the IMU 360 of FIG. 3C, wherein the IMU 360-1 of FIG. 3C may be positioned in a housing of the first driving module 30a, and the IMU 360 of FIG. 3C may be positioned in a housing of the second driving module 30b. The IMU 360-1 of FIG. 3C may obtain motion information (e.g., the 3-axis acceleration and rotation angle) of the right hip joint, and the IMU 360 of FIG. 3C may obtain motion information (e.g., the 3-axis acceleration and rotation angle) of the left hip joint. As still another example, the IMU 360-1 of FIG. 3C may be positioned in the first fastening portion 40a, and the IMU 360 of FIG. 3C may be positioned in the second fastening portion 40b. The IMU 360-1 of FIG. 3C may obtain motion information (e.g., the 3-axis acceleration and rotation angle) of the right knee (or the right knee joint), and the IMU 360 of FIG. 3C may obtain motion information (e.g., the 3-axis acceleration and rotation angle) of the left knee (or the left knee joint).

FIGS. 3A to 3C are block diagrams illustrating examples of a configuration of a wearable device according to an example embodiment.

According to an embodiment, a wearable device 300 of FIG. 3A may include the processor 310, angle sensors 320 and 320-1, a battery 330, a power management integrated circuit (PMIC) 340, a memory 350, an IMU (or an IMU sensor) 360, motor driver circuits 370 and 370-1, motors 380 and 380-1 (e.g., the first and second actuators described with reference to FIG. 2A), and a communication module 390.

The plurality of angle sensors 320 and 320-1, the plurality of motor driver circuits 370 and 370-1, and the plurality of motors 380 and 380-1 are shown in FIG. 3A, but these are merely an example. As another example, as shown in FIG. 3B, the wearable device 300-1 may include one angle sensor 320, one motor driver circuit 370, and one motor 380. As another example, as shown in FIG. 3C, the wearable device 300-2 may include the plurality of IMUs 360 and 360-1. The motion information obtained by the IMUs 360 and 360-1 of FIG. 3C may be used, for example, to determine the respective directions of both knees. Also, according to implementation, the wearable devices 300, 300-1, and 300-2 may include a plurality of processors. The number of motor driver circuits, the number of motors, or the number of processors may vary depending on a body part on which the wearable devices 300, 300-1, and 300-2 are worn.

The wearable device 300 of FIG. 3A, the wearable device 300-1 of FIG. 3B, and the wearable device 300-2 of FIG. 3C may be examples of the wearable device 110 and the wearable device 200.

According to an embodiment, the angle sensor 320, the motor driver circuit 370, and the motor 380 may be included in the first driving module 30a of FIG. 2A, and the angle sensor 320-1, the motor driver circuit 370-1, and the motor 380-1 may be included in the second driving module 30b of FIG. 2A.

According to an embodiment, the angle sensor 320 and the angle sensor 320-1 may each correspond to a Hall sensor, but are not limited thereto.

According to an embodiment, the angle sensor 320 may measure or sense at least one of the angle, angular velocity, or angular acceleration of a first joint (e.g., the left hip joint, etc.) of the user. The angle sensor 320 may transmit the measurement result (e.g., at least one of the angle value, angular velocity value, or angular acceleration value of the first joint) to the processor 310. For example, the angle sensor 320 may obtain the angular acceleration value by measuring the angular acceleration of the left hip joint angle of the user, and transmit the obtained angular acceleration value to the processor 310. Embodiments are not limited thereto, and the angle sensor 320 may obtain the angle value or angular velocity value by measuring the angle or angular velocity of the left hip joint angle of the user, and transmit the obtained angle value or angle velocity value to the processor 310. The processor 310 may calculate the angular acceleration value of the left hip joint angle through the angle value or angular velocity value received from the angle sensor 320.

According to an embodiment, the angle sensor 320-1 may measure or sense at least one of the angle, angular velocity, or angular acceleration of a second joint (e.g., the right hip joint) of the user. The angle sensor 320-1 may transmit the measurement result (e.g., at least one of the angle value, angular velocity value, or angular acceleration value of the second joint) to the processor 310. For example, the angle sensor 320-1 may obtain the angular acceleration value by measuring the angular acceleration of the right hip joint angle of the user, and transmit the obtained angular acceleration value to the processor 310. Embodiments are not limited thereto, and the angle sensor 320-1 may obtain the angle value or angular velocity value by measuring the angle or angular velocity of the right hip joint angle of the user, and transmit the obtained angle value or angle velocity value to the processor 310. The processor 310 may calculate the angular acceleration value of the right hip joint angle through the angle value or angular velocity value received from the angle sensor 320-1.

According to an embodiment, the angle sensor 320 and the angle sensor 320-1 may additionally measure the knee angles and ankle angles of the user according to the positions of the angle sensor 320 and the angle sensor 320-1.

According to an embodiment, the wearable devices 300, 300-1, and 300-2 may include a potentiometer. The potentiometer may sense an R-axis joint angle, an L-axis joint angle, an R-axis joint angular velocity, and an L-axis joint angular velocity according to a walking motion of the user. In this example, the R and L axes may be reference axes for the right leg and the left leg of the user, respectively. For example, the R/L axis may be set to be vertical to the ground and set such that a front side of a body of a person has a negative value and a rear side of the body has a positive value.

According to an embodiment, the PMIC 340 may charge the battery 330 using power supplied from an external power source. For example, the external power source and the wearable devices 300, 300-1, and 300-2 may be connected through a cable (e.g., a universal serial bus (USB) cable, etc.). The PMIC 340 may receive power from the external power source through the cable, and charge the battery 330 using the received power. According to embodiments, the PMIC 340 may charge the battery 330 through a wireless charging method.

According to an embodiment, the PMIC 340 may transmit the power stored in the battery 330 to the components (e.g., the processor 310, the angle sensors 320 and 320-1, the memory 350, the IMU 360, the motors 380 and 380-1, etc.) in the wearable devices 300, 300-1, and 300-2. The PMIC 340 may, for example, adjust the power stored in the battery 330 to a voltage or current level suitable for the components in the wearable device 300. The PMIC 340 may include, for example, a converter (e.g., a direct current (DC)-DC converter) or a regulator (e.g., a low-dropout (LDO) regulator or a switching regulator) configured to perform the adjustment described above.

According to an embodiment, the PMIC 340 may determine state information (e.g., a state of charge, a state of health, an overvoltage, a low voltage, an overcurrent, an overcharge, an overdischarge, an overheating, a short circuit, or a swelling) of the battery 330, and transmit the state information of the battery 330 to the processor 310. The processor 310 may control to provide the state information of the battery 330 to the user. For example, the processor 310 may output the state information of the battery 330 through at least one of a sound output module, a vibration output module, or a display module described below. For example, the processor 310 may transmit the state information of the battery 330 to the smart phone 120 through the communication module 390, and the smart phone 120 may display the state information of the battery 330 on the display.

According to an embodiment, the IMU 360 may obtain or measure motion information (or posture information) of the user. For example, the IMU 360 may measure or obtain 3-axis (e.g., x-axis, y-axis, and z-axis) accelerations and rotation angles (e.g., roll, pitch, and yaw) according to a walking motion of the user. The IMU 360 may transmit the obtained motion information (e.g., the measured 3-axis accelerations and rotation angles) to the processor 310.

According to an embodiment, the processor 310 may control the overall operation of the wearable devices 300, 300-1, and 300-2.

According to an embodiment, the processor 310 may, for example, control the components (e.g., the motor driver circuits 370 and 370-1, etc.) in the wearable devices 300, 300-1, and 300-2 by executing software (e.g., a program or instructions) stored in the memory 350, and perform various data processing or computation. As at least a portion of the data processing or computation, the processor 310 may store data received from other components (e.g., the IMU 360, the angle sensors 320 and 320-1, etc.) in the memory 350, and process the instructions or data stored in the memory 350.

According to an embodiment, the processor 310 may determine torque values for generating torques of the motors 380 and 380-1, and control the driving module 30 (e.g., the motor driver circuits 370 and 370-1) based on the determined torque values. For example, the processor 310 may determine a state factor *y*(*t*) indicating a state of a motion of the user according to an equation *y*(*t*) = sin(*q*_*r*(*t*)) - sin(*q_l*(*t*)). *q_l*(*t*) may denote the angle value of the first joint (e.g., the left hip joint), and *q_r*(*t*) may denote the angle value of the second joint (e.g., the right hip joint). The processor 310 may determine a torque value *τ*(*t*) according to an equation *τ*(*t*) = *κy*(*t -*△*t*). A gain *k* may be a parameter indicating the magnitude and direction of a torque generated by each of the motors 380 and 380-1. As the value of the gain *κ* increases, a greater torque may be output. If the gain *κ* is negative, a torque (or a resistance torque) acting as a resistance force may be output to the user, and if the gain *κ* is positive, a torque (or an assistance torque) acting as an assistance force may be output to the user. A delay *Δt* may be a parameter associated with a torque output timing. The value of the gain *κ* and the value of the delay *Δt* may be, for example, preset, and may be adjustable by the user, the wearable device 300, or the smart phone 120 paired with the wearable device 300. The processor 310 may determine a torque value *τᵣ*(*t*) for generating a torque from the motor 380-1 according to an equation *τᵣ*(*t*) *= τ*(*t*), and determine a torque *τₗ*(*t*) for generating a torque from the motor 380 according to *τₗ*(*t*) *= -τ*(*t*).

"Based on" as used herein covers based at least on
According to an embodiment, the motor driver circuits 370 and 370-1 may control the motors 380 and 380-1 based on the torque values received from the processor 310, and the motors 380 and 380-1 may generate torques by this control.

According to an embodiment, the communication module 390, comprising communication circuitry, may support the establishment of a direct (or wired) communication channel or a wireless communication channel between the wearable device 300, 300-1, or 300-2 and an external electronic device, and support the communication through the established communication channel. The communication module 390 may include one or more communication processors configured to support direct (or wired) communication or wireless communication. According to an embodiment, the communication module 390 may include a wireless communication module (e.g., a cellular communication module, a short-range wireless communication module, or a global navigation satellite system (GNSS) communication module) or a wired communication module (e.g., a local area network (LAN) communication module or a power line communication (PLC) module). A corresponding one of these communication modules may communicate with the external electronic device via a first network (e.g., a short-range communication network such as Bluetooth^{™}, wireless-fidelity (Wi-Fi) direct, or infrared data association (IrDA)) or a second network (e.g., a legacy cellular network, a 5G network, a next-generation communication network, the Internet, or a computer network). These various types of communication modules may be implemented as a single component (e.g., a single chip), or may be implemented as multiple components (e.g., multiple chips) separate from each other.

According to an embodiment, the wearable devices 300, 300-1, and 300-2 may include a display module. The display module may include, for example, a display and/or a lighting unit (e.g., the lighting unit 60 of FIG. 2A). The processor 310 may control the display module so that the display module may provide visual feedback to the user.

According to an embodiment, the wearable devices 300, 300-1, and 300-2 may include a sound output module. The sound output module may include, for example, a speaker. The processor 310 may control the sound output module so that the sound output module may provide auditory feedback to the user.

According to an embodiment, the wearable devices 300, 300-1, and 300-2 may include a vibration output module. The vibration output module may include, for example, a vibration motor. The processor 310 may control the vibration output module so that the vibration output module may provide tactile feedback (or haptic feedback) to the user.

According to an embodiment, at least one or all of the processor 310, the battery 330, the PMIC 340, the memory 350, the IMU 360, or the communication module 390 may be positioned in the base body 10 described above.

In the example shown in FIG. 3C, the wearable device 300-2 may include the plurality of IMUs 360 and 360-1. Embodiments are not limited thereto, and the wearable device 300-2 may include one IMU 360. At this time, the wearable device 300-2 may include one or more sensors (e.g., an ultra-wideband (UWB) radar sensor, a time of flight (ToF) sensor, a global positioning system (GPS), etc.) capable of measuring position (or distance). For example, each of the sensors capable of measuring position may be arranged in the wearable device 300-2 to measure the respective positions (or directions) of both knees of the user.

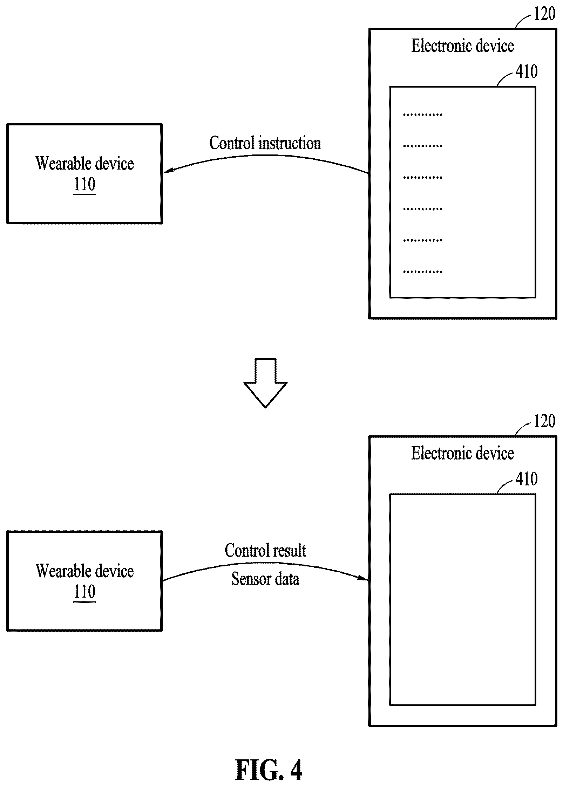

FIG. 4 is a diagram illustrating an interaction between a wearable device and an electronic device according to an example embodiment.

Referring to FIG. 4, the wearable device 110 may communicate with the smart phone 120 (e.g., a smart phone or smart watch). For example, the smart phone 120 may be a user terminal of the user who uses the wearable device 110 or a controller device dedicated to the wearable device 110. According to an embodiment, the wearable device 110 and the smart phone 120 may be connected to each other through short-range wireless communication (e.g., Bluetooth^{™} or Wi-Fi communication).

According to an embodiment, the smart phone 120 may verify a state of the wearable device 110 or execute an application to control or operate the wearable device 110. A screen of a user interface (UI) may be displayed to control an operation of the wearable device 110 on a display 410 of the smart phone 120 through the execution of the application. The UI may be, for example, a graphical user interface (GUI).

According to an embodiment, the user may input an instruction to control an operation of the wearable device 110 through the GUI screen on the display 410 of the smart phone 120 (e.g., an instruction to instruct the wearable device 110 to operate in an assistance mode of generating an assistance force or an instruction to instruct the wearable device 110 to operate in a resistance mode of generating a resistance force) or change settings of the wearable device 110. The smart phone 120 may generate a control instruction (or control signal) corresponding to an operation control instruction or a setting change instruction input by the user and transmit the generated control instruction to the wearable device 110. The wearable device 110 may operate according to the received control instruction and transmit a control result according to the control instruction and/or sensor data measured by the sensor (e.g., the angle sensors 320 and 320-1 and/or the IMU 360) of the wearable device 110 to the smart phone 120. The smart phone 120 may provide the user with result information (e.g., walking ability information, exercise ability information, or exercise posture evaluation information) derived by analyzing the control result and/or the sensor data through the GUI screen.

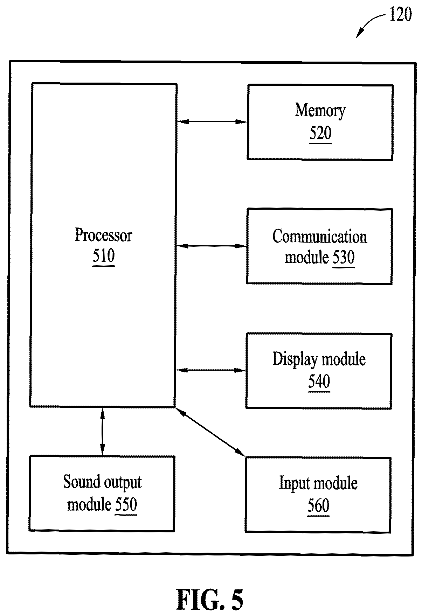

FIG. 5 is a diagram illustrating an example of a configuration of an electronic device according to an example embodiment.

Referring to FIG. 5, the smart phone 120 may include a processor 510, a memory 520, a communication module 530, a display module 540, a sound output module 550, and an input module 560. In an embodiment, at least one (e.g., the sound output module 550) of these components may be omitted from the smart phone 120, or one or more other components (e.g., a sensor module, a haptic module, and a battery) may be added thereto.

The processor 510 may control at least one other component (e.g., a hardware or software component) of the smart phone 120, and may perform a variety of data processing or computation. According to an embodiment, as at least part of data processing or computation, the processor 510 may store instructions or data received from another component (e.g., the communication module 530) in the memory 520, process the instructions or data stored in the memory 520, and store result data obtained as a result of processing in the memory 520.

According to an embodiment, the processor 510 may include a main processor (e.g., a central processing unit (CPU) or an application processor (AP)) or an auxiliary processor (e.g., a graphics processing unit (GPU)), a neural processing unit (NPU), an image signal processor (ISP), a sensor hub processor, or a communication processor (CP)) that is operable independently of, or in conjunction with the main processor.

The memory 520 may store a variety of data used by at least one component (e.g., the processor 510 or the communication module 530) of the smart phone 120. The data may include, for example, a program (e.g., an application), and input data or output data for instructions related thereto. The memory 520 may include at least one instruction executable by the processor 510. The memory 520 may include a volatile memory or a non-volatile memory.

The communication module 530 may support the establishment of a direct (e.g., wired) communication channel or a wireless communication channel between the smart phone 120 and another electronic device (e.g., the wearable device 110, the other wearable device 220, or the server 230), and support the communication through the established communication channel. The communication module 530 may include a communication circuit configured to perform a communication function. The communication module 530 may include one or more communication processors that are operable independently from the processor 510 (e.g., the application processor) and support direct (e.g., wired) communication or wireless communication. According to an embodiment, the communication module 530 may include a wireless communication module configured to perform wireless communication (e.g., a Bluetooth communication module, a cellular communication module, a Wi-Fi communication module, or a GNSS communication module) or a wired communication module (e.g., a LAN communication module or a power line communication (PLC) module). For example, the communication module 530 may transmit a control instruction to the wearable device 110 and receive, from the wearable device 110, at least one of sensor data including body motion information of the user who is wearing the wearable device 110, state data of the wearable device 110, or control result data corresponding to the control instruction.

The display module 540 may visually provide information to the outside (e.g., the user) of the smart phone 120. The display module 540 may include, for example, a liquid-crystal display (LCD) or organic light-emitting diode (OLED) display, a hologram device, or a projector device. The display module 540 may further include a control circuit configured to control the driving of a display. In an embodiment, the display module 540 may further include a touch sensor set to sense a touch or a pressure sensor set to sense the intensity of a force generated by the touch. The display module 540 may output a user interface screen for controlling the wearable device 110 or providing a variety of information (e.g., exercise evaluation information and setting information of the wearable device 110).

The sound output module 550 may output sound signals to the outside of the smart phone 120. The sound output module 550 may include a speaker configured to play back a guiding sound signal (e.g., an operation start sound or an operation error alarm), music content, or a guiding voice based on the state of the wearable device 110. For example, when it is determined that the wearable device 110 is not normally worn on the body of the user, the sound output module 550 may output a guiding voice for informing the user is wearing the wearable device 110 abnormally or for guiding the user to wear the wearable device 110 normally.

The input module 560 may receive a command or data to be used by a component (e.g., the processor 510) of the smart phone 120 from the outside (e.g., the user) of the smart phone 120. The input module 560 may include an input component circuit and may receive a user input. The input module 560 may include, for example, a touch recognition circuit for recognizing a touch on a key (e.g., a button) and/or a screen.

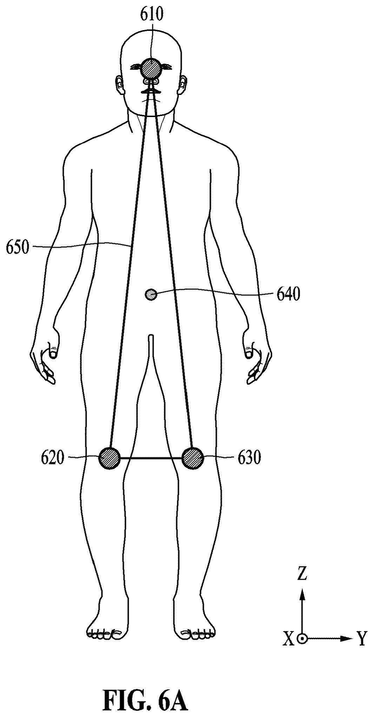

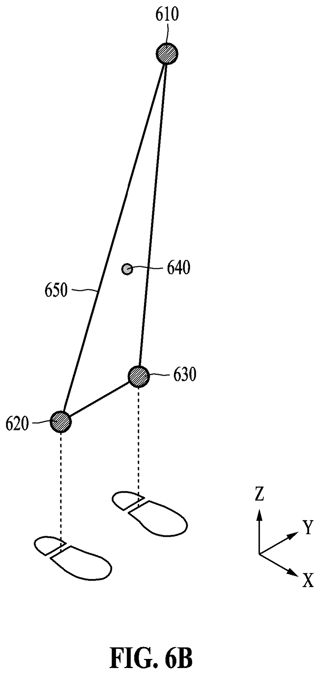

FIGS. 6A and 6B are diagrams illustrating an example of determining a posture of a standing user by a wearable device according to an example embodiment.

An example of a triangle set by the wearable device 110 (e.g., the wearable device 300-2) is shown in FIGS. 6A and 6B.

According to an embodiment, the wearable device 110 may request a user to look at the front and stand motionlessly.

According to an embodiment, the wearable device 110 may include the plurality of IMUs (or IMU sensors) 360 and 360-1. The IMU 360 of FIG. 3C may be arranged in the wearable device 110 to be positioned, for example, near the left knee of the user, and the IMU 360-1 of FIG. 3C may be arranged in the wearable device 110 to be positioned, for example, near the right knee of the user. The motion information obtained by the IMU 360 may be used by the processor 310, for example, to determine the direction of the left knee (or the direction of a left step), and the motion information obtained by the IMU 360-1 may be used by the processor 310, for example, to determine the direction of the right knee (or the direction of a right step).

Although not shown in FIG. 6A, the user may wear the other wearable device 130 (e.g., the wireless earphones 131 and/or the smart glasses 133). The other wearable device 130 may be wirelessly connected to, for example, the wearable device 110. As another example, the other wearable device 130 may be wirelessly connected to the wearable device 110 through the smart phone 120.

According to an embodiment, the other wearable device 130 may set a first point (or a head point) 610 corresponding to a part (e.g., the head) of the body of the user, and transmit a coordinate value (or a position value) (e.g., a three-dimensional coordinate value) of the first point 610 to the wearable device 110. For example, the other wearable device 130 may include a sensor capable of measuring distance (or position), and a coordinate value (or a position value) (e.g., a three-dimensional coordinate value) of the other wearable device 130 may be calculated through sensing data from the sensor capable of measuring distance (or position). The other wearable device 130 may set a point of the calculated coordinate value as the first point 610, and transmit the coordinate value of the first point 610 to the wearable device 110. The sensor capable of measuring distance (or position) may include, for example, an IMU, an acceleration sensor, a UWB radar sensor, a ToF sensor, a GPS, and the like, but is not limited thereto.

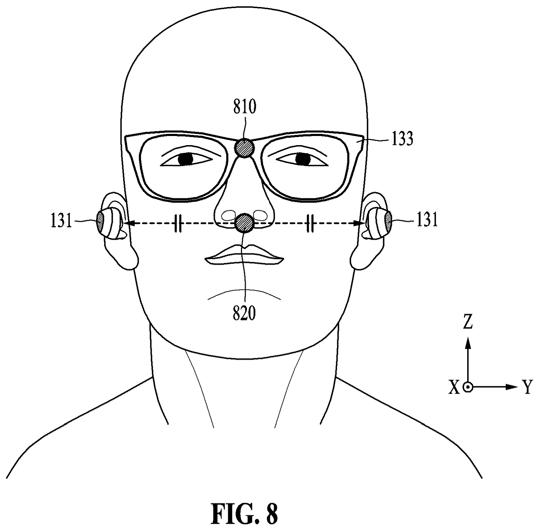

According to another embodiment, the wearable device 110 may receive the sensing data obtained by the sensor capable of measuring distance (or position), from the other wearable device 130. The wearable device 110 (e.g., the processor 310) may calculate the coordinate value (or the position value) (e.g., three-dimensional coordinate value) of the other wearable device 130 based on the sensing data received from the other wearable device 130, and set the point of the calculated coordinate value as the first point 610. Setting the first point 610 is described below with reference to FIG. 8.

According to an embodiment, the IMU 360-1 may obtain acceleration information (e.g., 3-axis acceleration) and/or rotation angle information (e.g., roll, pitch, and yaw) of the right knee. The processor 310 of the wearable device 110 may receive the acceleration information (e.g., the 3-axis acceleration) and/or the rotation angle information (e.g., the roll, pitch, and yaw) of the right knee from the IMU 360-1, and calculate a coordinate value (or a position value) corresponding to the position of the right knee (or the position of the IMU 360-1) through the acceleration information and/or the rotation angle information received from the IMU 360-1. The processor 310 may set a point of a coordinate value corresponding to the position of the right knee (or the position of the IMU 360-1) as a second point (or a right knee point) 620.

According to an embodiment, the IMU 360 may obtain acceleration information (e.g., 3-axis acceleration) and/or rotation angle information (e.g., roll, pitch, and yaw) of the left knee. The processor 310 of the wearable device 110 may receive the acceleration information (e.g., the 3-axis acceleration) and/or the rotation angle information (e.g., the roll, pitch, and yaw) of the left knee from the IMU 360, and calculate a coordinate value (or a position value) corresponding to the position of the left knee (or the position of the IMU 360) through the acceleration information and/or the rotation angle information received from the IMU 360. The processor 310 may set a point of a coordinate value corresponding to the position of the left knee (or the position of the IMU 360) as a third point (or a left knee point) 630.

According to an embodiment, the processor 310 may connect the first point 610, the second point 620, and the third point 630 to each other to form a triangle 650. The processor 310 may form the triangle 650 by static modeling (e.g., setting and connecting the points 610, 620, and 630). The triangle 650 may be set for a static user (or a user standing motionlessly) and thus, may be represented as a static triangle.

According to an embodiment, in part (e.g., the base body 10 or the main belt 50) of the wearable device 110, the origin 640 may be set (or determined) to calculate relative coordinate values of the points 610, 620, and 630. The coordinate value of the origin 640 may be (Xo, Yo, Zo) and may be fixed. The processor 310 may calculate the relative coordinate values of the first to third points 610, 620, and 630. For example, the coordinate value of the first point 610 may be (X₁, Y₁, Z₁), the coordinate value of the second point 620 may be (X₂, Y₂, Z₂), and the coordinate value of the third point 630 may be (X₃, Y₃, Z₃). The processor 310 may calculate the relative coordinate value (X₁-Xo, Y₁-Yo, Z₁-Zo) of the first point 610, calculate the relative coordinate value (X₂-Xo, Y₂-Yo, Z₂-Zo) of the second point 620, and calculate the relative coordinate value (X₃-Xo, Y₃-Yo, Z₃-Zo) of the third point 630.

According to an embodiment, the processor 310 may determine the posture of the user based on the points 610, 620, and 630. For example, the processor 310 may determine that the head posture of the user is an abnormal head posture (e.g., a turtle neck posture) in response to the determination that the first point 610 is a predetermined level ahead of a line between the second point 620 and the third point 630. For example, a first axis direction (e.g., the X-axis direction) may be determined to be the front direction of the user. The processor 310 may determine that the head posture of the user is the abnormal head posture (e.g., the turtle neck posture) when the difference between a first axis value (e.g., the X-axis value X₁-Xo) of the relative coordinate value of the first point 610 and a first axis value (e.g., the X-axis value X₂-Xo) of the relative coordinate value of the second point 620 is greater than or equal to a predetermined level in the first axis direction and/or when the difference between the first axis value (e.g., the X-axis value X₁-Xo) of the relative coordinate value of the first point 610 and a first axis value (e.g., the X-axis value X₃-Xo) of the relative coordinate value of the third point 630 is greater than or equal to the predetermined level in the first axis direction. The processor 310 may determine that the head posture of the user is a normal head posture when the difference between a first axis value (e.g., the X-axis value X₁-Xo) of the relative coordinate value of the first point 610 and a first axis value (e.g., the X-axis value X₂-Xo) of the relative coordinate value of the second point 620 is less than the predetermined level and/or when the difference between the first axis value (e.g., the X-axis value X₁-Xo) of the relative coordinate value of the first point 610 and a first axis value (e.g., the X-axis value X₃-Xo) of the relative coordinate value of the third point 630 is less than the predetermined level.

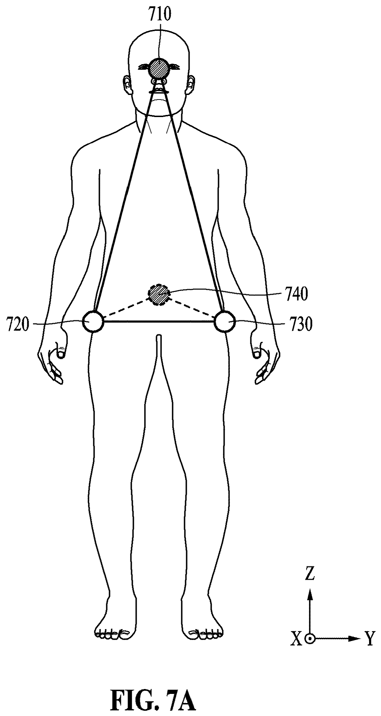

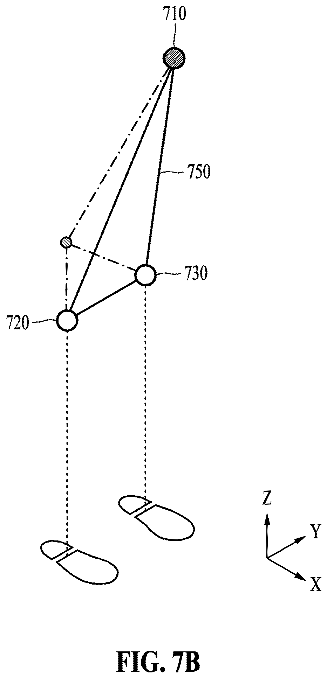

FIGS. 7A and 7B are diagrams illustrating another example of determining a posture of a standing user by a wearable device according to an example embodiment.

An example of a triangle set by the wearable device 110 (e.g., the wearable device 300 or the wearable device 300-1) is shown in FIGS. 7A and 7B.

According to an embodiment, the wearable device 110 may request a user to look at the front and stand motionlessly.

According to an embodiment, the wearable device 110 may include the IMU 360 of FIGS. 3A and 3B. The IMU 360 of FIGS. 3A and 3B may be positioned, for example, in a base body (e.g., the base body 10 of FIG. 2A) of the wearable device 110.

Although not shown in FIG. 7A, the user may wear the other wearable device 130 (e.g., the wireless earphones 131 and/or the smart glasses 133). The other wearable device 130 may be wirelessly connected to, for example, the wearable device 110. As another example, the other wearable device 130 may be wirelessly connected to the wearable device 110 through the smart phone 120.

According to an embodiment, the other wearable device 130 may set a first point (or a head point) 710 corresponding to a part (e.g., the head) of the body of the user, and transmit a coordinate value (or a position value) (e.g., a three-dimensional coordinate value) of the first point 710 to the wearable device 110. For example, the other wearable device 130 may include a sensor capable of measuring distance (or position), and a coordinate value (or a position value) of the other wearable device 130 may be calculated through sensing data of the sensor capable of measuring distance (or position). The other wearable device 130 may set a point of the calculated coordinate value as the first point 710, and transmit the coordinate value of the first point 710 to the wearable device 110.

According to another embodiment, the wearable device 110 may receive the sensing data obtained by the sensor capable of measuring distance (or position), from the other wearable device 130. The wearable device 110 (e.g., the processor 310) may calculate the coordinate value (or the position value) of the other wearable device 130 based on the sensing data received from the other wearable device 130, and set the point of the calculated coordinate value as the first point 710. Setting the first point 710 is described below with reference to FIG. 8.

According to an embodiment, the IMU 360 may obtain acceleration information (e.g., 3-axis acceleration) and/or rotation angle information (e.g., roll, pitch, and yaw) of the user. The processor 310 of the wearable device 110 may receive the acceleration information (e.g., the 3-axis acceleration) and/or the rotation angle information (e.g., the roll, pitch, and yaw) of the use from the IMU 360, and calculate a coordinate value (or a position value) corresponding to the position of the base body 10 (or the position of the IMU 360) through the acceleration information and/or the rotation angle information received from the IMU 360. The processor 310 may set a point of a coordinate value corresponding to the position of the base body 10 (or the position of the IMU 360) as a back point 740.

According to an embodiment, the processor 310 may be aware in advance of how far each of the first driving module 30a positioned near the left hip joint of the user and the second driving module 30b positioned near the right hip joint of the user is apart from the base body 10. For example, the processor 310 may be aware in advance that the first driving module 30a is apart from the base body 10 by (Xa, Ya, Za) and that the second driving module 30b is apart from the base body 10 by (Xb, Yb, Zb). The processor 310 may calculate a coordinate value corresponding to the position of the second driving module 30b (or the right hip joint) through the coordinate value of the back point 740 and (Xb, Yb, Zb) and determine a point of the coordinate value corresponding to the position of the second driving module 30b (or the right hip joint) to be a second point 720. The processor 310 may calculate a coordinate value corresponding to the position of the first driving module 30a (or the left hip joint) through the coordinate value of the back point 740 and (Xa, Ya, Za) and determine a point of the coordinate value corresponding to the position of the first driving module 30a (or the left hip joint) to be a third point 730.

According to an embodiment, the processor 310 may connect the first point 710, the second point 720, and the third point 730 to each other to form a triangle 750. The processor 310 may form the triangle 750 by static modeling (e.g., setting and connecting the points 710, 720, and 730). The triangle 750 may be set for a static user (or a user standing motionlessly) and thus, may be represented as a static triangle.

According to an embodiment, the processor 310 may determine the posture of the user based on the points 710, 720, and 730. For example, the processor 310 may determine that the head posture of the user is an abnormal head posture (e.g., a turtle neck posture) in response to the determination that the first point 710 is a predetermined level ahead of a line between the second point 720 and the third point 730. For example, the processor 310 may determine that the head posture of the user is the abnormal head posture (e.g., the turtle neck posture) when the difference between a first axis value (e.g., the X-axis value) of the coordinate value of the first point 710 and a first axis value (e.g., the X-axis value) of the coordinate value of the second point 720 is greater than or equal to a predetermined level and/or when the difference between the first axis value (e.g., the X-axis value) of the coordinate value of the first point 710 and a first axis value (e.g., the X-axis value) of the coordinate value of the third point 730 is greater than or equal to the predetermined level. The processor 310 may determine that the head posture of the user is the normal head posture when the difference between the first axis value (e.g., the X-axis value) of the coordinate value of the first point 710 and the first axis value (e.g., the X-axis value) of the coordinate value of the second point 720 is less than the predetermined level and/or when the difference between the first axis value (e.g., the X-axis value) of the coordinate value of the first point 710 and the first axis value (e.g., the X-axis value) of the coordinate value of the third point 730 is less than the predetermined level.

FIG. 8 is a diagram illustrating an example of setting a first point of a user by a wearable device according to an example embodiment.

Referring to FIG. 8, a user may wear the wireless earphones 131 and/or the smart glasses 133. The wearable device 110 (e.g., the wearable device 300, the wearable device 300-1, or the wearable device 300-2) may be wirelessly connected to the wireless earphones 131 and/or the smart glasses 133. Embodiments are not limited thereto, and the wearable device 110 may be wirelessly connected to the wireless earphones 131 and/or the smart glasses 133 through the smart phone 120.

According to an embodiment, the wireless earphones 131 may include a sensor (e.g., an acceleration sensor) capable of measuring position. The wireless earphones 131 may transmit sensing data (e.g., 3-axis acceleration information) from the acceleration sensor to the wearable device 110. The wearable device 110 (e.g., the processor 310) may calculate respective coordinate values of the wireless earphones 131 through the sensing data received from the wireless earphones 131. The wearable device 110 (e.g., the processor 310) may set a point 820 of the average value of the respective coordinate values of the wireless earphones 131 as a first point (e.g., the first point 610 or the first point 710).

According to an embodiment, the smart glasses 133 may include a sensor (e.g., an IMU, etc.) capable of measuring position. The smart glasses 133 may transmit sensing data from the sensor capable of measuring position to the wearable device 110. The wearable device 110 (e.g., the processor 310) may calculate a coordinate value of the smart glasses 133 through the sensing data received from the smart glasses 133. The wearable device 110 (e.g., the processor 310) may set a point 810 of the coordinate value of the smart glasses 133 as a first point (e.g., the first point 610 or the first point 710).

According to an embodiment, the user may be wearing both the wireless earphones 131 and the smart glasses 133. In this case, the wearable device 110 may set the point 810 of the coordinate value of the smart glasses 133 as the first point (e.g., the first point 610 or the first point 710).

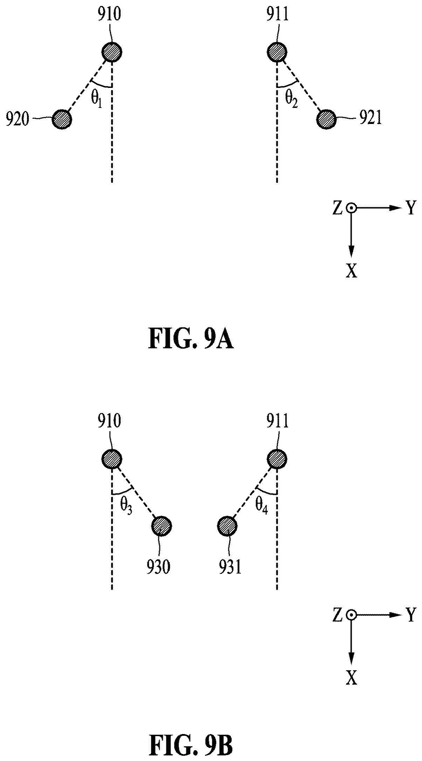

FIGS. 9A and 9B are diagrams illustrating examples of a calibration operation of a wearable device according to an example embodiment.

Referring to FIGS. 9A and 9B, in the wearable device 110 (e.g., the wearable device 300, the wearable device 300-1, or the wearable device 300-2), a reference point 910 may be set to determine the direction of a second point 920 or 930 (e.g., the second point 620 or the second point 720), and a reference point 911 may be set to determine the direction of a third point 921 or 931 (e.g., the third point 630 or the third point 730). The reference points 910 and 911 may correspond to, for example, both knees of the user, respectively. The reference points 910 and 911 may have fixed coordinate values. The coordinate value of the reference point 910 may be (X_{R}, Y_{R}, Z_{R}), and the coordinate value of the reference point 911 may be (X_{L}, Y_{L}, Z_{L}).

In the examples shown in FIGS. 9A and 9B, the processor 310 of the wearable device 110 may calculate the coordinate value of the second point (e.g., the second point 920 of FIG. 9A or the second point 930 of FIG. 9B) through acceleration information and/or rotation angle information received from the IMU 360-1, and calculate the coordinate value of the third point (e.g., the third point 921 of FIG. 9A or the third point 931 of FIG. 9B) through acceleration information and/or rotation angle information received from the IMU 360. The processor 310 may be aware of the direction of the second point (e.g., the second point 920 of FIG. 9A or the second point 930 of FIG. 9B) from the reference point 910 and the direction of the third point (e.g., the third point 921 of FIG. 9A or the third point 931 of FIG. 9B) from the reference point 911.

The processor 310 may determine the front direction of the user through the direction of the second point 920 or 930 from the reference point 910 and the direction of the third point 921 or 931 from the reference point 911, calibrate the respective coordinate values of the reference point 910 and the reference point 911 such that the determined front direction is a reference (e.g., a reference that is the basis for determining a gait posture), and calibrate the respective coordinate values of the second point 920 or 930 and the third point 921 or 931. For example, the processor 310 may calculate a vector between the reference point 910 and the second point 920 or 930, calculate a vector between the reference point 911 and the third point 921 or 931, calculate a mean vector of the vector between the reference point 910 and the second point 920 or 930 and the vector between the reference point 911 and the third point 921 or 931, and determine a direction of the calculated mean vector to be the front direction of the user. In the examples shown in FIGS. 9A and 9B, the processor 310 may determine the X-axis direction to be the front direction of the user. The processor 310 may calibrate the respective coordinate values of the reference point 910, the reference point 911, the second point 920 or 930, and the third point 921 or 931 such that the determined front direction (e.g., the X-axis direction) is a reference (e.g., a reference that is the basis for determining a gait posture of the user).

According to an embodiment, in the example shown in FIG. 9A, the processor 310 may calculate the angle (e.g., θ₁ of FIG. 9A) between a line of the front direction (e.g., the X-axis direction) of the reference point 910 (hereinafter, referred to as the "first reference line") and a line of the second point 920. The line of the second point 920 may be, for example, a line generated by projecting a line between the reference point 910 and the second point 920 onto a first plane (e.g., the XY plane). The processor 310 may calculate the angle (e.g., θ₂ of FIG. 9A) between a line in the front direction of the reference point 911 (hereinafter, referred to as the "second reference line") and a line of the third point 921. The line of the third point 921 may be, for example, a line generated by projecting a line between the reference point 910 and the third point 921 onto the first plane (e.g., the XY plane). When it is checked through the calculated angles (e.g., θ₁ and θ₂) that the line of the second point 920 and the line of the third point 921 are spread apart, the processor 310 may determine that the user is standing in the front direction with both feet pointing outward.

According to an embodiment, in the example shown in FIG. 9B, the processor 310 may calculate the angle (e.g., θ₃ of FIG. 9B) between a first reference line and the line of the second point 930 and calculate the angle (e.g., θ₄ of FIG. 9B) between a second reference line and the line of the third point 931. When it is checked through the calculated angles (e.g., θ₃ and θ₄) that the line of the second point 930 and the line of the third point 931 point inward, the processor 310 may determine that the user is standing in the front direction with both feet pointing inward.

In the embodiment described below, the coordinate value of a second point (e.g., a second point 1020 described later) and the coordinate value of a third point (e.g., a third point 1030 described later) may be calibrated coordinate values.

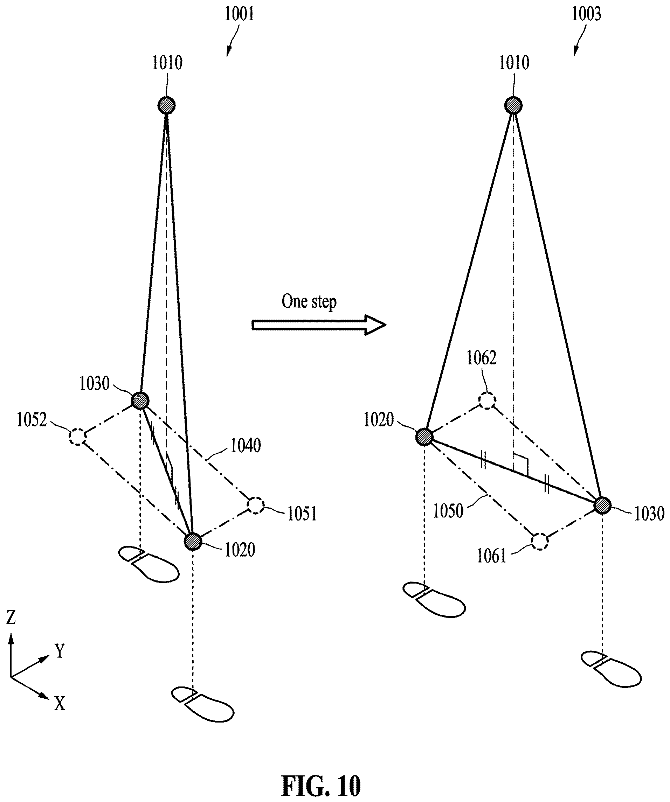

FIG. 10 is a diagram illustrating an example of determining a posture of a moving user by a wearable device according to an example embodiment.

In the example shown in FIG. 10, the X-axis direction may be the front direction of a user, and the user may perform, for example, a walking exercise.

In the structure of a human body, the knees and feet are connected, and the knees and feet may point in the same direction. According to an embodiment, the wearable device 110 (e.g., the wearable device 300, the wearable device 300-1, or the wearable device 300-2) may determine the directions of the feet (or the gait) and the gait posture of the user through the directions of the knees of the user (e.g., the directions of the second point 1020 and the third point 1030 described later). The wearable device 110 may analyze the directions of the feet (or the gait) and the gait posture of the user through the directions of the knees (e.g., the directions of the second point 1020 and the third point 1030 described later).

According to an embodiment, the wearable device 110 (e.g., the wearable device 300, the wearable device 300-1, or the wearable device 300-2) may calculate factors for determining the posture (e.g., the head posture, gait posture, etc.) of the user. The factors for determining the posture of the user may include, for example, at least one of a vector (or relative coordinate value) of a first point 1010 (e.g., the first point 610 or the first point 710), a vector (or relative coordinate value) of a second point 1020 (e.g., the second point 620 or the second point 720), a vector (or relative coordinate value) of a third point 1030 (e.g., the third point 630 or the third point 730), a polygon (e.g., a quadrangle) with the second point 1020 and the third point 1030 as diagonal points, or the step length of the user. The polygon with the second point 1020 and the third point 1030 as diagonal points may be used, for example, to determine whether the user is walking.

According to an embodiment, the processor 310 of the wearable device 110 may receive sensing data sensed by the other wearable device 130 worn by the user from the other wearable device 130. The processor 310 may track the first point 1010 in real time using the sensing data received from the other wearable device 130, and determine the vector (or relative coordinate value) of the first point 1010 by tracking the first point 1010. The processor 310 may calculate the coordinate value (or the position value) of the first point 1010 based on the sensing data received from the other wearable device 130. The processor 310 may determine the magnitude and direction from the origin 640 to the coordinate value of the first point 1010 to be the vector of the first point 1010, or calculate the difference between the coordinate value of the first point 1010 and the origin 640 as the relative coordinate value of the first point 1010.

According to an embodiment, the processor 310 may calculate the vectors (or relative coordinate values) of the second point 1020 and the third point 1030 through an IMU (e.g., the IMU 360 and/or the IMU 360-1).

For example, the IMU 360 and the IMU 360-1 may be arranged in the wearable device 110 so as to be positioned near both knees of the user, respectively. The processor 310 may track the second point 1020 using motion information (e.g., acceleration information and/or rotation angle of the right knee) received from the IMU 360-1, and determine the vector (or relative coordinate value) of the second point 1020 in real time by tracking the second point 1020. The processor 310 may calculate the coordinate value (or the position value) of the second point 1020 through the motion information (e.g., the acceleration information and/or rotation angle of the right knee) received from the IMU 360-1. The processor 310 may determine the magnitude and direction from the origin 640 to the coordinate value of the second point 1020 to be the vector of the second point 1020, or calculate the difference between the coordinate value of the second point 1020 and the origin 640 as the relative coordinate value of the second point 1020. The processor 310 may track the third point 1030 using motion information (e.g., acceleration information and/or rotation angle of the left knee) received from the IMU 360, and determine the vector (or relative coordinate value) of the third point 1030 in real time by tracking the third point 1030. The processor 310 may calculate the coordinate value (or the position value) of the third point 1030 through the motion information (e.g., the acceleration information and/or rotation angle of the left knee) received from the IMU 360. The processor 310 may determine the magnitude and direction from the origin 640 to the coordinate value of the third point 1030 to be the vector of the third point 1030, or calculate the difference between the coordinate value of the third point 1030 and the origin 640 as the relative coordinate value of the third point 1030.

As another example, the IMU 360 may be arranged inside the base body 10 of the wearable device 110 so as to be positioned near the back of the user. The processor 310 may track the second point 1020 and the third point 1030 using motion information (e.g., acceleration information and/or rotation angle of the user) received from the IMU 360, and determine the respective vectors (or relative coordinate values) of the second point 1020 and the third point 1030 in real time by tracking the second point 1020 and the third point 1030. The processor 310 may determine the respective coordinate values (or position values) of the second point 1020 and the third point 1030 through the motion information (e.g., the acceleration information and/or rotation angle of the user) received from the IMU 360. The processor 310 may determine the magnitude and direction from the origin 640 to the coordinate value of the second point 1020 to be the vector of the second point 1020, or calculate the difference between the coordinate value of the second point 1020 and the origin 640 as the relative coordinate value of the second point 1020. The processor 310 may determine the magnitude and direction from the origin 640 to the coordinate value of the third point 1030 to be the vector of the third point 1030, or calculate the difference between the coordinate value of the third point 1030 and the origin 640 as the relative coordinate value of the third point 1030.

According to an embodiment, the processor 310 may form a polygon (e.g., a quadrangle) with the second point 1020 and the third point 1030 as diagonal points. If the user is standing motionlessly, the X-axis value of the second point 1020 and the X-axis value of the third point 1030 may be almost the same, so that a polygon with the second point 1020 and the third point 1030 as diagonal points may not be formed. If the user moves, the second point 1020 and the third point 1030 may have different X-axis values, so that a polygon with the second point 1020 and the third point 1030 as diagonal points may be formed. If the user moves, the processor 310 may set a point 1051 or 1061 which is a predetermined distance (e.g., the distance between the knees) apart from the second point 1020 on the Y-axis and a point 1052 or 1062 which is the predetermined distance (e.g., the distance between the knees) apart from the third point 1030 on the Y-axis, and form a quadrangle through the second point 1020, the third point 1030, the point 1051 or 1061 which is the predetermined distance apart from the second point 1020 on the Y-axis, and the point 1052 or 1062 which is the predetermined distance apart from the third point 1030 on the Y-axis. The processor 310 may determine that the user is walking (or moving) when a polygon with the second point 1020 and the third point 1030 as diagonal points is formed, and determine that the user is not walking when a polygon with the second point 1020 and the third point 1030 as diagonal points is not formed.

According to an embodiment, the processor 310 may calculate the distance between the second point 1020 and the third point 1030 (hereinafter, referred to as the "first distance"). The processor 310 may determine that the user is walking when the first distance changes, and may determine that the user is not walking when the first distance does not change. In other words, the processor 310 may determine that the user is standing and not walking, when the first distance does not change.

According to an embodiment, the processor 310 may determine whether the foot of the user touches the ground through the IMU 360 and/or the IMU 360-1. For example, when the left foot touches the ground, an impact may be applied to the left foot, and the impact may cause noise in the motion information obtained by the IMU 360. When noise caused by the impact on the left foot is found from the motion information received from the IMU 360, the processor 310 may determine that the left foot touches the ground and determine (or calculate) the timepoint at which the left foot touches the ground. When the right foot touches the ground, an impact may be applied to the right foot, and the impact may cause noise in the motion information obtained by the IMU 360-1. When noise caused by the impact on the right foot is found from the motion information received from the IMU 360-1, the processor 310 may determine that the right foot touches the ground and determine (or calculate) the timepoint at which the right foot touches the ground. Depending on the implementation, the wearable device 110 may include one IMU 360. In this case, the processor 310 may determine whether the right foot touches the ground or the left foot touches the ground, using at least one of the motion information obtained through the IMU 360 or the angle values or angular velocity values obtained by the angle sensors 320 and 320-1.

According to an embodiment, the processor 310 may calculate the walking speed of the user through the motion information from the IMU 360 and/or the IMU 360-1, calculate the difference between the timepoint at which the right foot touches the ground and the timepoint at which the left foot touches the ground, and determine the step length (e.g., the length of one step) by multiplying the calculated difference by the calculated walking speed.

According to an embodiment, the processor 310 may determine the walking state of the user. The walking state may include, for example, a first state in which the user is standing, a second state in which the user walks in place, a third state in which the rear foot touches the ground and the front foot is in the air, and a fourth state in which the rear foot and the front foot touch the ground.

For example, the processor 310 may form a quadrangle 1040 through the second point 1020, the third point 1030, the point 1051 which is the predetermined distance apart from the second point 1020 on the Y-axis, and the point 1052 which is the predetermined distance apart from the third point 1030 on the Y-axis, and determine the walking state of the user to be the fourth state (e.g., a state 1001 of FIG. 10), when a touch of the front foot (e.g., the right foot) with the ground is detected through the IMU 360-1 while the rear foot (e.g., the left foot) is touching the ground.

For example, the processor 310 may determine the walking state of the user to be the third state, when the quadrangle 1040 is formed and a touch of the front foot (e.g., the right foot) with the ground is not detected through the IMU 360-1 while the rear foot (e.g., the left foot) is touching the ground.

For example, the processor 310 may determine the walking state of the user to be the second state, when it is detected through the IMUs 360 and 360-1 that an axis value (e.g., the X-axis value) corresponding to the front direction in the respective coordinate values of the second point 1020 and the third point 1030 does not change and that both feet alternately touch the ground.

For example, the processor 310 may determine the walking state of the user to be the first state, when the motion information received from the IMU 360 or 360-1 does not change.

When the user moves in the state 1001, the first point 1010, the second point 1020, and the third point 1030 may move. When the user moves, the processor 310 may track the first point 1010 using the sensing data received from the other wearable device 130, and track the second point 1020 and the third point 1030 through the motion information obtained by the IMU 360 and/or the IMU 360-1. The processor 310 may determine (or update) in real time the respective vectors (or relative coordinate values) of the first point 1010, the second point 1020, and the third point 1030 by tracking the first point 1010, the second point 1020, and the third point 1030. As the first point 1010, the second point 1020, and the third point 1030 may each move, a triangle formed through the first point 1010, the second point 1020, and the third point 1030 may change. A triangle that changes according to the motion of the user may be referred to as a "dynamic triangle".

A state 1003 may be a state in which the left foot of the user lands forward on the ground and the right foot of the user lands backward on the ground. The processor 310 may form a quadrangle 1050 through the second point 1020, the third point 1030, the point 1062 which is the predetermined distance apart from the second point 1020 on the Y-axis, and the point 1061 which is the predetermined distance apart from the third point 1030 on the Y-axis, and determine the walking state of the user to be the fourth state (e.g., the state 1003 of FIG. 10), when a touch of the front foot (e.g., the left foot) with the ground is detected through the IMU 360 while the rear foot (e.g., the right foot) is touching the ground.

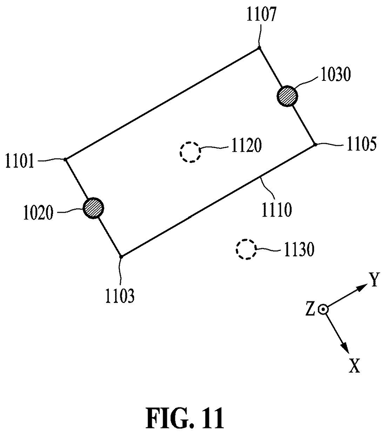

FIG. 11 is a diagram illustrating an example of determining a posture of a user by a wearable device according to an example embodiment.

In the example shown in FIG. 11, the X-axis direction may be the forward direction of a user.

According to an embodiment, the processor 310 of the wearable device 110 (e.g., the wearable device 300, the wearable device 300-1, or the wearable device 300-2) may form an area based on the second point 1020 and the third point 1030. The formed area may be, for example, the area used to determine whether the user is looking at the front (or gazing at the front). The processor 310 may project the first point 1010 onto the plane of the second point 1020 and the third point 1030 (e.g., the polygon described with reference to FIG. 10), and determine whether a point generated by projecting the first point 1010 (hereinafter, referred to as the "projection point") is positioned in the area formed based on the second point 1020 and the third point 1030. The processor 310 may determine that the user is looking at the front when the projection point is positioned in the formed area, and determine that the user is not looking at the front when the projection point is positioned out of the formed area.

An area 1110 formed based on the second point 1020 and the third point 1030 when the walking state of the user is the fourth state (e.g., the state 1001 of FIG. 10) is shown in FIG. 11. The area 1110 shown in FIG. 11 may be the area viewed from the Z-axis.

In the example shown in FIG. 11, the coordinate value (or relative coordinate value) of the second point 1020 may be (X₂, Y₂, Z₂), and the coordinate value (or relative coordinate value) of the third point 1030 may be (X₃, Y₃, Z₃).

The processor 310 may form the area 1110 using the second point 1020, the third point 1030, and preset values α and β. For example, the processor 310 may determine the two-dimensional coordinate value (X₂+α, Y₂+β) of a point 1103 by adding α and β to the X-axis value (e.g., X₂) and the Y-axis value (e.g., Y₂) of the second point 1020, respectively, and determine the two-dimensional coordinate value (X₂-α, Y₂-β) of a point 1101 by subtracting α and β from the X-axis value (e.g., X₂) and the Y-axis value (e.g., Y₂) of the second point 1020, respectively. The processor 310 may determine the two-dimensional coordinate value (X₃+α, Y₃+β) of a point 1105 by adding α and β to the X-axis value (e.g., X₃) and the Y-axis value (e.g., Y₃) of the third point 1030, respectively, and determine the two-dimensional coordinate value (X₃-α, Y₃-β) of a point 1107 by subtracting α and β from the X-axis value (e.g., X₃) and the Y-axis value (e.g., Y₃) of the third point 1030, respectively. The processor 310 may form the area 1110 through the points 1101, 1103, 1105, and 1107.

The coordinate value (or relative coordinate value) of the first point 1010 may be, for example, (X₁, Y₁, Z₁). The processor 310 may generate a projection point by projecting the first point 1010 onto a plane (e.g., the quadrangle 1040 of FIG. 10) formed by the second point 1020 and the third point 1030. The processor 310 may determine whether the projection point of the first point 1010 is positioned in the area 1110. The processor 310 may determine whether the X-axis value of the projection point of the first point 1010 belongs to a first range (e.g., X₃-α < ~ < X₂+α), and determine whether the Y-axis value of the projection point of the first point 1010 belongs to a second range (e.g., Y₂-β < ~ < Y₃+β).

The processor 310 may determine that the user is looking at the front when the projection point of the first point 1010 is positioned in the area 1110. The processor 310 may determine that the user is looking at the front when the X-axis value of the projection point of the first point 1010 belongs to the first range (e.g., X₃-α < ~ < X₂+α) and the Y-axis value of the projection point of the first point 1010 belongs to the second range (e.g., Y₂-β < ~ < Y₃+β). A point 1120 shown in FIG. 11 may be an example of the projection point of the first point 1010. The projection point 1120 may be positioned in the area 1110. The X-axis value of the projection point 1120 may belong to the first range (e.g., X₃-α < ~ < X₂+α), and the Y-axis value of the projection point 1120 may belong to the second range (e.g., Y₂-β < ~ < Y₃+β). In this case, the processor 310 may determine that the user is looking at the front.

The processor 310 may determine that the user is not looking at the front when the projection point of the first point 1010 is positioned out of the area 1110 (or not positioned in the area 1110). The processor 310 may determine that the user is not looking at the front when the X-axis value of the projection point of the first point 1010 does not belong to the first range (e.g., X₃-α < ~ < X₂+α) and/or the Y-axis value of the projection point of the first point 1010 does not belong to the second range (e.g., Y₂-β < ~ < Y₃+β). A point 1130 shown in FIG. 11 may be another example of the projection point 1120 of the first point 1010. The projection point 1130 may be positioned not in the area 1110, but out of the area 1110. The X-axis value of the projection point 1130 may belong to the first range (e.g., X₃-α < ~ < X₂+α), but the Y-axis value of the projection point 1130 may not belong to the second range (e.g., Y₂-β < ~ < Y₃+β). In this case, the processor 310 may determine that the user is not looking at the front.

According to an embodiment, the processor 310 may determine that the user is walking while looking at the front, when it is detected that the user is walking (e.g., the walking state of the user is the third state or the fourth state) and it is determined that the projection point of the first point 1010 is positioned in the area 1110 formed based on the second point 1020 and the third point 1030. The processor 310 may determine that the user is walking while not looking at the front when it is detected that the user is walking and it is determined that the projection point of the first point 1010 is not positioned in the area 1110 formed based on the second point 1020 and the third point 1030.

According to an embodiment, the processor 310 may determine the walking state of the user to be the fourth state (e.g., the state 1001 or the state 1003 of FIG. 10). The processor 310 may calculate a first distance (e.g., the distance between the second point 1020 and the third point 1030). The processor 310 may calculate the distance between one of the second point 1020 and the third point 1030 (e.g., a point corresponding to the front foot) and the projection point of the first point 1010 (hereinafter, referred to as the "second distance"). The processor 310 may determine the head posture of the user to be a normal head posture when the difference between n times (e.g., "0.5" times) the calculated first distance and the calculated second distance is within a predetermined range. The processor 310 may determine the head posture of the user to be an abnormal head posture (e.g., a turtle neck posture) when the difference between n times (e.g., "0.5" times) the calculated first distance and the calculated second distance is beyond the predetermined range. n is not limited to *"0.5",* and depending on the implementation, n may correspond to a number other than "0.5" (e.g., "0.4", "0.6", etc.).

For example, the processor 310 may determine the walking state of the user to be the state 1001 of FIG. 10. The processor 310 may determine the walking state of the user to be a state in which the rear foot (e.g., the left foot) and the front foot (e.g., the right foot) of the user touch the ground. The processor 310 may calculate the distance between the second point 1020 and the third point 1030 (e.g., the first distance). The projection point of the first point 1010 may be the point 1120 of FIG. 11. The processor 310 may calculate the second distance between the projection point 1120 of the first point 1010 and the second point 1020 corresponding to the front foot (e.g., the right foot). The processor 310 may determine the head posture of the user to be a normal head posture when the difference between n times the calculated first distance and the calculated second distance is within the predetermined range. The processor 310 may determine the head posture of the user to be an abnormal head posture when the difference between n times the calculated first distance and the calculated second distance is beyond the predetermined range. According to embodiments, the processor 310 may determine the head posture of the user to be a first abnormal head posture (e.g., the turtle neck posture or a forward head posture) when the difference between n times the calculated first distance and the calculated second distance is less than the predetermined range, and determine the head posture of the user to be a second abnormal head posture (e.g., a backward head posture that the head is positioned backward) when the difference between n times the calculated first distance and the calculated second distance exceeds the predetermined range.

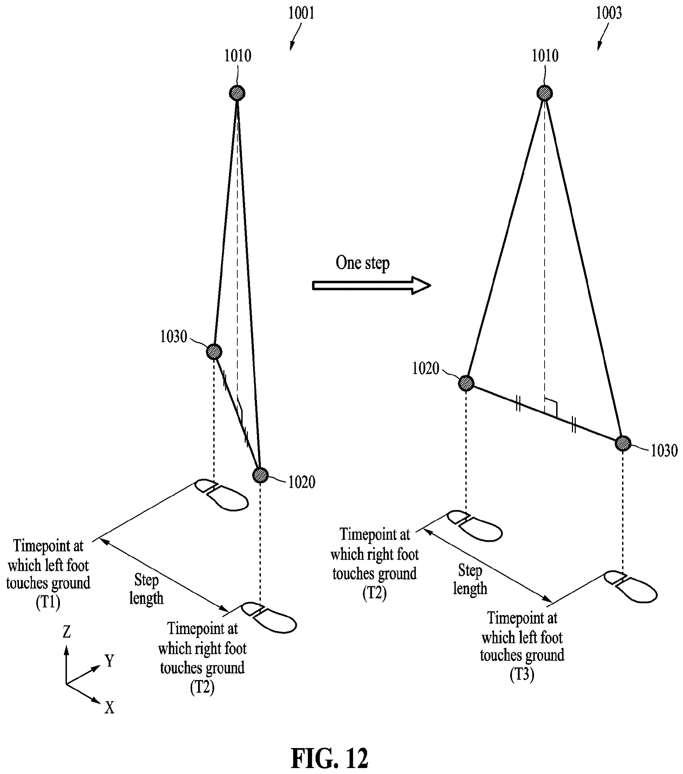

FIG. 12 is a diagram illustrating an example of determining a gait posture of a user by a wearable device according to an example embodiment.

In the example shown in FIG. 12, the processor 310 of the wearable device 110 (e.g., the wearable device 300, the wearable device 300-1, or the wearable device 300-2) may determine that the left foot touches the ground based on the motion information received from the IMU 360, and determine (or calculate) the timepoint (e.g., a timepoint T1 of FIG. 12) at which the left foot touches the ground. The processor 310 may determine that the right foot touches the ground based on the motion information received from the IMU 360-1, and determine (or calculate) the timepoint (e.g., a timepoint T2 of FIG. 12) at which the right foot touches the ground.

In the example shown in FIG. 12, the processor 310 may calculate the walking speed of the user through the motion information obtained by the IMU 360 and/or the IMU 360-1. The processor 310 may calculate the difference (e.g., T2-T1) between the timepoint (e.g., T2) at which the right foot touches the ground and the timepoint (e.g., T1) at which the left foot touches the ground, and determine the step length (e.g., the length of one step) by multiplying the calculated difference (e.g., T2-T1) by the calculated walking speed.

After one step of the user, the processor 310 may determine that the left foot touches the ground based on the motion information received from the IMU 360, and determine (or calculate) the timepoint (e.g., a timepoint T3 of FIG. 12) at which the left foot touches the ground. The processor 310 may calculate the difference (e.g., T3-T2) between the timepoint (e.g., T3) at which the left foot touches the ground and the timepoint (e.g., T2) at which the right foot touches the ground, and determine the step length (e.g., the length of one step) by multiplying the calculated difference (e.g., T3-T2) by the calculated walking speed.

According to an embodiment, the processor 310 may determine the direction of the third point 1030 at the timepoint (e.g., T1 and/or T3) at which the left foot touches the ground, and determine the gait posture of the user based on the direction of the third point 1030. For example, the processor 310 may determine the coordinate value of the third point 1030 through the motion information received from the IMU 360 at the timepoint (e.g., T1 and/or T3) at which the left foot touches the ground. The processor 310 may calculate the vector of the third point 1030 using the coordinate value of the third point 1030 and the coordinate value (or calibrated coordinate value) of a reference point (e.g., the reference point 911 described with reference to FIGS. 9A and 9B). The processor 310 may calculate the angle between the vector of the third point 1030 and a line (e.g., the second reference line described with reference to FIGS. 9A and 9B) in the front direction. The angle between the vector of the third point 1030 and the line (e.g., the second reference line) in the front direction may denote, for example, the gait angle of the left foot.

According to an embodiment, the processor 310 may determine the direction of the second point 1020 at the timepoint (e.g., T2) at which the right foot touches the ground. For example, the processor 310 may determine the coordinate value of the second point 1020 through the motion information received from the IMU 360-1 at the timepoint (e.g., T2) at which the right foot touches the ground. The processor 310 may calculate the vector of the third point 1030 using the coordinate value of the second point 1020 and the coordinate value (or calibrated coordinate value) of the reference point (e.g., the reference point 910 described with reference to FIGS. 9A and 9B), and calculate the angle between the vector of the third point 1030 and the line (e.g., the first reference line described with reference to FIGS. 9A and 9B) in the front direction. The angle between the vector of the third point 1030 and the line (e.g., the first reference line) in the front direction may denote, for example, the gait angle of the right foot.

According to an embodiment, the processor 310 may determine the gait posture of the user to be the first abnormal gait posture (e.g., an out-toeing posture) in response to the determination that the angle between the vector of the third point 1030 and the line in the front direction (e.g., the gait angle of the left foot) and/or the angle between the vector of the second point 1020 and the line in the front direction (e.g., the gait angle of the right foot) exceeds a predetermined range (e.g., "0" to "15" degrees) toward the outside of the front direction. The processor 310 may determine the gait posture of the user to be the second abnormal gait posture (e.g., an in-toeing posture) in response to the determination that the angle between the vector of the third point 1030 and the line in the front direction (e.g., the gait angle of the left foot) and/or the angle between the vector of the second point 1020 and the line in the front direction (e.g., the gait angle of the right foot) exceeds the predetermined range (e.g., "0" to "15" degrees) toward the inside of the front direction.

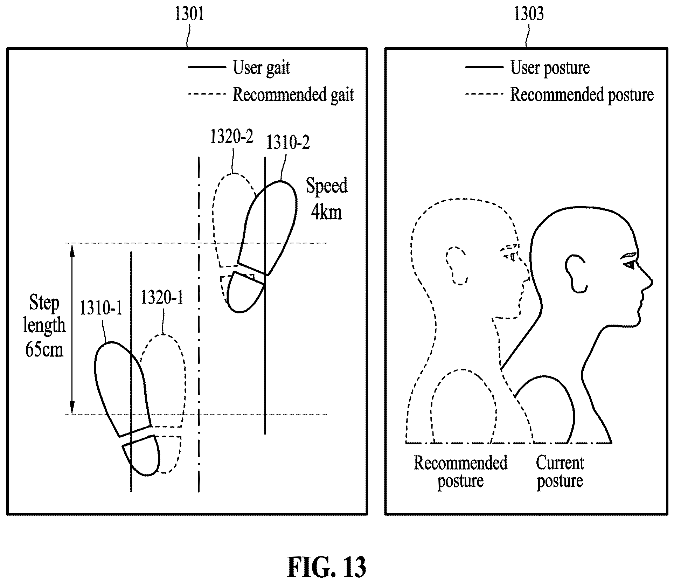

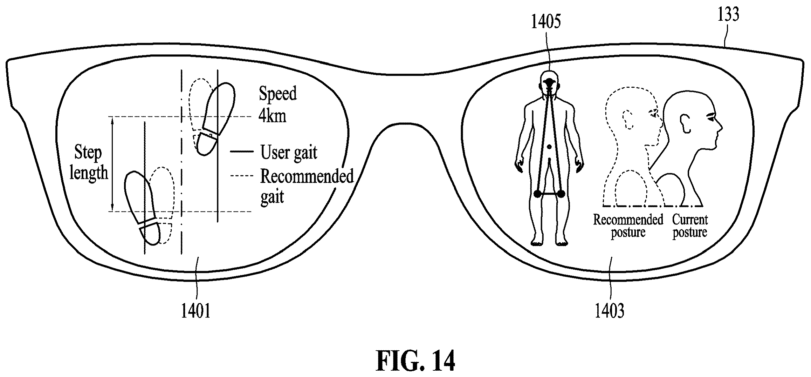

FIGS. 13 and 14 are diagrams illustrating examples of screens in which a posture of a user is displayed according to an example embodiment.

In the example shown in FIG. 13, according to an embodiment, an electronic device (e.g., at least one of the smart phone 120, the smart watch 132, or the smart glasses 133) may display a screen 1301 including objects 1310-1 and 1310-2 representing the gait (or gait posture) of a user and/or objects 1320-1 and 1320-2 representing a recommended gait (or recommended gait posture). The electronic device may display the screen 1301 visualizing the gait of the user and the recommended gait. The electronic device may link with the wearable device 110 to provide the user with the gait (or gait posture) of the user and/or the recommended gait (or recommended gait posture) in real time.

According to an embodiment, the electronic device may display a screen 1303 including an object representing the head posture of the user and/or an object representing a recommended head posture on a display. The electronic device may display the screen 1303 visualizing the head posture of the user and the recommended head posture on the display. The electronic device may link with the wearable device 110 to provide the user with the head posture of the user and/or the recommended head posture in real time.

According to an embodiment, the processor 310 of the wearable device 110 (e.g., the wearable device 300, the wearable device 300-1, or the wearable device 300-2) may transmit information about the head posture of the user (e.g., whether it is a normal head posture or an abnormal head posture (e.g., a first abnormal head posture or a second abnormal head posture)) to the electronic device. The processor 310 of the wearable device 300-2 may control the electronic device to display the recommended head posture when the head posture of the user is an abnormal head posture. The electronic device may display the screen 1303 including the head posture of the user on the display using the information about the head posture of the user. The screen 1303 including the recommended head posture may be displayed. The electronic device may provide the user with the head posture of the user and/or the recommended head posture through the screen 1303.

According to an embodiment, when the front foot of the user is in the air (e.g., when the walking state of the user is determined to be a third state), the processor 310 of the wearable device 110 (e.g., the wearable device 300, the wearable device 300-1, or the wearable device 300-2) may predict the gait in the front direction of a point (e.g., the second point 1020 or the third point 1030) corresponding to the front foot, and determine the predicted gait to be the recommended gait (or recommended gait posture) of the front foot. The processor 310 may control the electronic device to provide the user with the recommended gait (or recommended gait posture). The processor 310 may transmit a control signal indicating that the recommended gait (or recommended gait posture) is to be displayed to the electronic device. The electronic device may display an object (e.g., the object 1320-1 and/or the object 1320-2 of FIG. 13) representing the recommended gait (or recommended gait posture) on the display. The processor 310 may control the electronic device to provide the user with the gait (or gait posture) of the front foot when the front foot of the user touches the ground. When the front foot of the user touches the ground, the processor 310 may transmit information about the gait (or gait posture) of the front foot (e.g., the gait angle and/or whether the user is walking with the foot pointing outward (or inward)) to the electronic device. The electronic device may display an object (e.g., the object 1310-1 and/or the object 1310-2 of FIG. 13) for the gait of the front foot on the display using the information about the gait (or gait posture) of the front foot.

For example, when the right foot of the user touches the rear surface and the left foot of the user is in the air as the front foot, the processor 310 may predict the gait in the front direction of the third point 1030 corresponding to the front foot (e.g., the left foot), and determine the predicted gait to be the recommended gait of the left foot. The processor 310 may transmit a control signal indicating that the recommended gait (or recommended gait posture) of the left foot is to be displayed to the electronic device. The electronic device may display the object 1320-1 for the recommended gait of the left foot on the display. As shown in FIG. 13, the object 1320-1 for the recommended gait of the left foot may point in the front direction. When the left foot of the user touches the ground, the processor 310 may transmit information about the gait (or gait posture) of the left foot (e.g., the gait angle and/or whether the user is walking with the left foot pointing outward (or inward)) to the electronic device. The electronic device may display the object 1310-1 for the gait of the left foot on the display using the information about the gait (or gait posture) of the left foot. When the user is walking with the left foot pointing outward, the object 1310-1 for the gait of the left foot may point outward, as shown in FIG. 13.

When the left foot of the user touches the rear surface and the right foot of the user is in the air as the front foot, the processor 310 may predict the gait in the front direction of the second point 1020 corresponding to the front foot (e.g., the right foot), and determine the predicted gait to be the recommended gait of the right foot. The processor 310 may transmit a control signal indicating that the recommended gait (or recommended gait posture) of the right foot is to be displayed to the electronic device. The electronic device may display the object 1320-2 for the recommended gait of the right foot on the display. As shown in FIG. 13, the object 1320-2 for the recommended gait of the right foot may point in the front direction. When the right foot of the user touches the ground, the processor 310 may transmit information about the gait (or gait posture) of the right foot (e.g., the gait angle and/or whether the user is walking with the right foot pointing outward (or inward)) to the electronic device. The electronic device may display the object 1310-2 for the gait of the right foot on the display using the information about the gait (or gait posture) of the right foot. When the user is walking with the right foot pointing outward, the object 1310-2 for the gait of the right foot may point outward, as shown in FIG. 13.

As described above, the processor 310 may calculate the walking speed (e.g., 4 km/h of FIG. 13) and step length (e.g., 65 cm of FIG. 13) of the user, and transmit the calculated step length and walking speed to the electronic device. The electronic device may display the step length and walking speed of the user on the display.

In the example shown in FIG. 14, the wearable device 110 may be wirelessly connected directly to the smart glasses 133 or through the smart phone 120.

According to an embodiment, the processor 310 of the wearable device 110 may transmit a control signal indicating that the recommended gait (or recommended gait posture) is to be displayed to the smart glasses 133. The processor 310 may transmit information about the gait of the user (e.g., the gait angle and/or whether the user is walking with the foot pointing outward (or inward) to the smart glasses 133. The processor 310 may transmit walking indices (e.g., the walking speed and/or step length) of the user to the smart glasses 133.

According to an embodiment, the smart glasses 133 may generate an AR image by including at least one of the objects 1310-1 and 1310-2 for the gait of the user, the objects 1320-1 and 1320-2 for the recommended gait, the object representing the head posture of the user, or the object representing the recommended head posture in an image captured through one or more front cameras, and display the AR image on displays 1401 and 1403. The smart glasses 133 may display at least one of the objects 1310-1 and 1310-2 for the gait of the user, the objects 1320-1 and 1320-2 for the recommended gait, the object representing the head posture of the user, or the object representing the recommended head posture on the image captured through the one or more front cameras.

As shown in the example of FIG. 14, the smart glasses 133 may display the objects 1310-1 and 1310-2 for the gait of the user and the objects 1320-1 and 1320-2 for the recommended gait on the first display 1401, and display the object representing the head posture of the user and the object representing the recommended head posture on the second display 1403. In the example shown in FIG. 14, the smart glasses 133 may display an object 1405 representing a human body on the second display 1403. This is an example, and according to embodiments, the smart glasses 133 may not display the object 1405. The smart glasses 133 may receive the walking indices (e.g., the step length and/or walking speed) of the user from the wearable device 110, and display the walking indices on at least one of the first display 1401 or the second display 1403.

According to an embodiment, the smart glasses 133 may receive a guide for the walking of the user from the wearable device 110, and display the received guide on at least one of the first display 1401 or the second display 1403. For example, when the walking speed of the user is fast, the wearable device 110 may transmit a guide to reduce the walking speed (e.g., a message saying "Please reduce the walking speed") to the smart glasses 133, and the smart glasses 133 may display the guide to reduce the walking speed on the first display 1401 and/or the second display 1403. When the step length of the user is wide, the wearable device 110 may transmit a guide to reduce the step length to the smart glasses 133, and the smart glasses 133 may display the guide to reduce the step length (e.g., a message saying "Please reduce the step length") on the first display 1401 and/or the second display 1403.

According to an embodiment, the wearable device 110 may be wirelessly connected directly to the wireless earphones 131 or through the smart phone 120. The processor 310 of the wearable device 110 may generate a first voice signal indicating a guide to the gait (or gait posture) of the user (e.g., "The user is currently out-toeing. Please walk with your toes pointing inward"), and transmit the first voice signal to the wireless earphones 131. The wireless earphones 131 may output the first voice signal. The processor 310 may generate a second voice signal indicating a guide for the head posture of the user, and transmit the generated second voice signal to the wireless earphones 131. The wireless earphones 131 may output the second voice signal received from the wearable device 110.

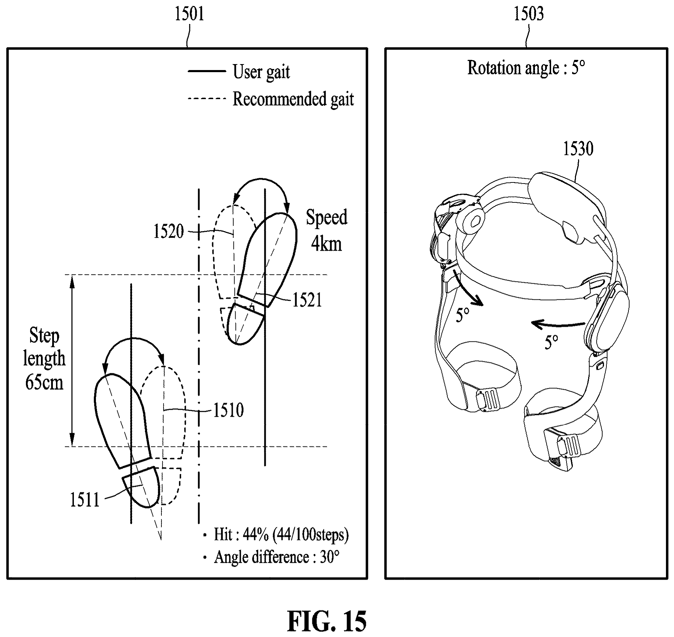

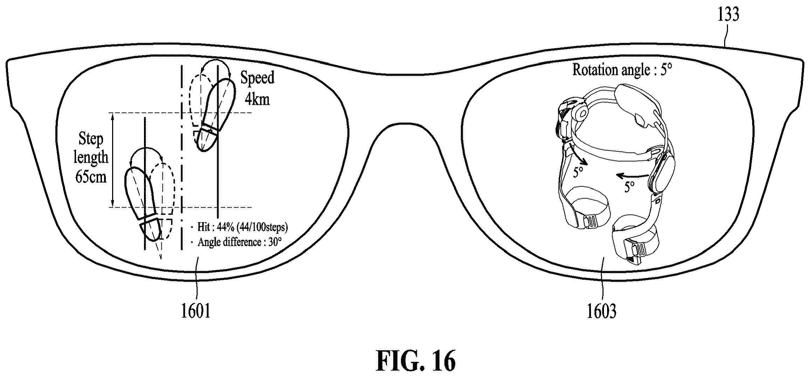

FIGS. 15 and 16 are diagrams illustrating examples of operations for correcting a posture of a user by a wearable device according to an example embodiment.

In the example shown in FIG. 15, according to an embodiment, an electronic device (e.g., at least one of the smart phone 120, the smart watch 132, or the smart glasses 133) may display a screen 1501 including objects 1310-1 and 1310-2 representing the gait of a user and/or objects 1320-1 and 1320-2 representing a recommended gait.

According to an embodiment, the electronic device may display a screen 1503 including an object 1530 of the wearable device 110. Arrows indicating that the driving module 30 of the wearable device 110 is to be rotated inward for the thighs to correct the gait posture of the user may be displayed on the screen 1503.

According to an embodiment, the processor 310 of the wearable device 110 (e.g., the wearable device 300, the wearable device 300-1, or the wearable device 300-2) may calculate the matching rate (e.g., the hit rate of FIG. 15) by which steps (e.g., "100" steps) of the user matches recommended steps.

For example, the processor 310 may calculate the angle between a line 1510 (e.g., a second reference line) indicating the direction of the recommended gait of the left foot and a line 1511 indicating the direction of the gait of the left foot (e.g., the gait angle of the left foot). The processor 310 may determine that the gait of the left foot matches the recommended gait of the left foot when the angle between the line 1510 and the line 1511 (e.g., the gait angle of the left foot) is less than a predetermined angle (e.g., "15" degrees), and determine that the gait of the left foot does not match the recommended gait when the angle between the line 1510 and the line 1511 (e.g., the gait angle of the left foot) exceeds the predetermined angle (e.g., "15" degrees). The processor 310 may calculate the angle between a line 1520 (e.g., a first reference line) indicating the direction of the recommended gait of the right foot and a line 1521 indicating the direction of the gait of the right foot (e.g., the gait angle of the right foot). The processor 310 may determine that the gait of the right foot matches the recommended gait of the right foot when the angle between the line 1520 and the line 1521 (e.g., the gait angle of the right foot) is less than a predetermined angle (e.g., "15" degrees), and determine that the gait of the right foot does not match the recommended gait of the right foot when the angle between the line 1520 and the line 1521 (e.g., the gait angle of the right foot) exceeds the predetermined angle (e.g., "15" degrees).

The processor 310 may calculate the number of steps, among a given number of steps (e.g., "100" steps), that match the recommended steps, and calculate the ratio between the given number and the calculated number as the matching rate (e.g., the hit rate of FIG. 15). In the example shown in FIG. 15, the processor 310 may determine that "44" steps, out of "100" steps, match the recommended step, and calculate the matching rate as 44%. The processor 310 may determine that the angle differences between the directions of the steps of the left foot, out of the given number of (e.g., "100") steps, and the direction of the recommended step of the left foot (e.g., the average value of the gait angles of the left foot) are, for example, "30" degrees on average, and determine that the angle differences between the directions of the steps of the right foot and the direction of the recommended step of the right foot (e.g., the average value of the gait angles of the right foot) are, for example, "30" degrees on average.

According to an embodiment, the processor 310 may transmit the angle differences (e.g., "30" degrees) and the matching rate (e.g., 44%) to the electronic device, and the electronic device may display the angle differences (e.g., "30" degrees) and the matching rate (e.g., 44%) on the display.

According to an embodiment, the processor 310 may determine whether the walking direction of the user is a straight forward direction through previous gait information of the user (or previous motion information obtained by the IMU 360 and the IMU 360-1). In response to the determination that the walking direction of the user is a straight forward direction, the processor 310 may perform an operation to correct the gait posture of the user. The operation to correct the gait posture may include, for example, an operation of controlling the driving module 30 to be inclined (or rotated) inward or outward, and/or an operation of controlling to output vibration in a pattern having directionality (e.g., a direction from the inside to the outside or a direction from the outside to the inside). In response to the determination that the walking direction of the user is not a straight forward direction, the processor 310 may defer performing an operation to correct the gait posture of the user.

According to an embodiment, the processor 310 may determine a rotation angle value indicating how much the driving module 30 is to be inclined (or rotated or tilted) based on at least one of the respective directions of the second point 620 and the third point 630 when the user is standing, the matching rate (e.g., 44%) calculated based on the number of matches of the steps of the user with the recommended steps, the duration of mismatches between the steps of the user and the recommended steps, or the angle difference (e.g., "30" degrees) between the steps and the recommended steps. In response to the determination that the walking direction of the user is a straight forward direction, the processor 310 may determine the rotation angle value indicating how much the driving module 30 is to be inclined.

According to an embodiment, when the gait posture of the user is determined to be a first abnormal gait posture (e.g., an out-toeing posture), the processor 310 may control the driving module 30 to be inclined (or rotated or tilted) inward by the determined rotation angle value. The processor 310 may transmit the determined rotation angle value to the electronic device. In the example shown in FIG. 15, the electronic device may display arrows indicating that the driving module 30 of the wearable device 110 is to be inclined (or rotated) inward by the rotation angle value (e.g., "5" degrees). For example, the angle formed between the first driving module 30a and a first axis (e.g., the X-axis) may be A degrees, and the angle formed between the second driving module 30b and the first axis (e.g., the X-axis) may be A degrees. When the first driving module 30a is inclined inward by the determined rotation angle value (e.g., "5" degrees) (or points toward the user), the angle formed between the first driving module 30a and the first axis (e.g., the X-axis) may be A+5 degrees. When the second driving module 30b is inclined inward by the determined rotation angle value (e.g., "5" degrees) (or points toward the user), the angle formed between the second driving module 30b and the first axis (e.g., the X-axis) may be A+5 degrees.

According to an embodiment, when the gait posture of the user is determined to be a second abnormal gait posture (e.g., an in-toeing posture), the processor 310 may control the driving module 30 to be inclined (or rotated or tilted) outward by the determined rotation angle value. The processor 310 may transmit the determined rotation angle value to the electronic device. The electronic device may display arrows indicating that the driving module 30 of the wearable device 110 is to be inclined (or rotated) outward by the rotation angle value (e.g., "5" degrees). For example, when the first driving module 30a is inclined outward by the determined rotation angle value (e.g., "5" degrees), the angle formed between the first driving module 30a and the first axis (e.g., the X-axis) may be A-5 degrees. When the second driving module 30b is inclined outward by the determined rotation angle value (e.g., "5" degrees), the angle formed between the second driving module 30b and the first axis (e.g., the X-axis) may be A-5 degrees.

According to an embodiment, the wearable device 110 may include a vibration module to output vibration. The vibration module may include one or more vibration motors. When the gait posture of the user is determined to be the first abnormal gait posture, the processor 310 may control the vibration module to output vibration in such a pattern as to move from the outside to the inside (e.g., from the outside of the thigh (or knee) to the inside). Such vibration may be output to the thigh (or knee) of the front leg, for example, when the front foot is in the air. When the gait posture of the user is determined to be the second abnormal gait posture, the processor 310 may control the vibration module to output vibration in such a pattern as to move from the inside to the outside (e.g., from the inside of the thigh (or knee) to the outside). Such vibration may be output to the thigh (or knee) of the front leg, for example, when the front foot is in the air. The processor 310 may control the vibration module to increase the intensity of vibration as the angle difference (e.g., "30" degrees) between the gait and the recommended gait increases.

In the example shown in FIG. 16, the wearable device 110 may be wirelessly connected directly to the smart glasses 133 or through the smart phone 120.

The processor 310 of the wearable device 110 may transmit, to the smart glasses 133, at least one of the matching rate (e.g., 44%) calculated based on the number of matches of the steps of the user with the recommended steps, the angle difference (e.g., "30" degrees) between the gait and the recommended gait, the walking speed, the step length, or the rotation angle value.

According to an embodiment, the smart glasses 133 may display at least one of the objects 1310-1 and 1310-2 for the gait of the user, the objects 1320-1 and 1320-2 for the recommended gait, the matching rate (e.g., 44%), the angle difference (e.g., "30" degrees), the walking speed, the step length, the rotation angle value, or the object 1530 of the wearable device 110 on an image captured through one or more front cameras. As shown in the example of FIG. 16, the smart glasses 133 may display the objects 1310-1 and 1310-2 for the gait of the user, the objects 1320-1 and 1320-2 for the recommended gait, the matching rate (e.g., 44%), the angle difference (e.g., "30" degrees), the step length, and the walking speed on a first display 1601, and display the rotation angle value and the arrows indicating the driving module 30 of the wearable device 110 is to be inclined inward on a second display 1603.

According to an embodiment, the smart glasses 133 may receive a guide for correcting the gait posture of the user from the wearable device 110, and display the received guide on at least one of the first display 1601 or the second display 1603. For example, when the gait posture of the user is determined to be the first abnormal gait posture, the wearable device 110 may transmit a guide for correcting the gait posture (e.g., a message saying "Please walk with your toes pointing inward") to the smart glasses 133, and the smart glasses 133 may display the guide for correcting the gait posture on the first display 1601 and/or the second display 1603.

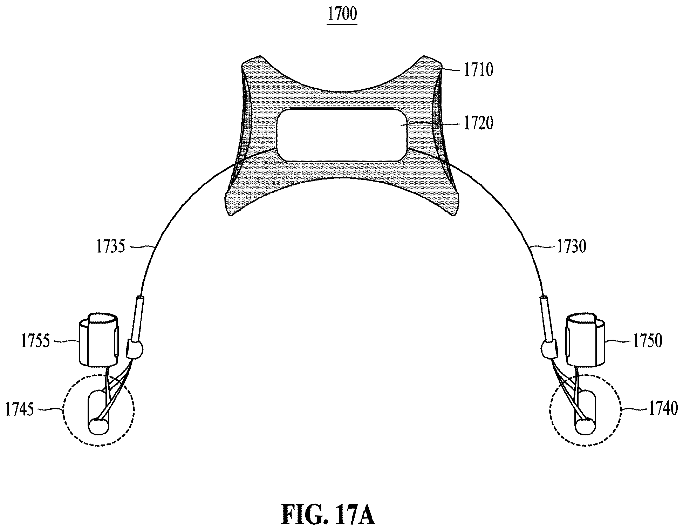

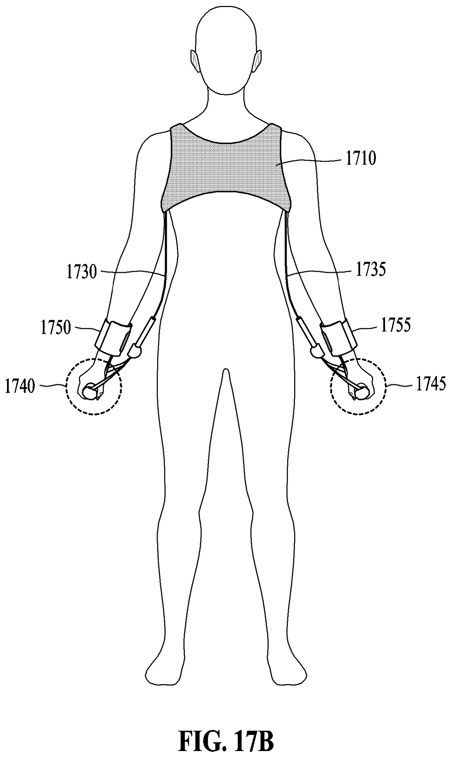

FIGS. 17A and 17B are diagrams illustrating an example of an upper body-type wearable device according to an example embodiment.

Referring to FIG. 17A, according to an embodiment, an upper body-type wearable device 1700 may include a wearable portion 1710, a control system 1720, cables 1730 and 1735, gripping parts 1740 and 1745, and wearable parts 1750 and 1755.

The wearable portion 1710 may be worn on the body of a user. For example, the wearable portion 1710 may be worn on the upper body of the user. The wearable portion 1710 may fit the upper body of the user and support a portion of the upper body of the user. A base plate of the wearable portion 1710 may be worn on the back of the user. For example, the base plate may have a flat plate shape. A front surface of the base plate may face the back of the user. A rear surface of the base plate may face the control system 1720.

According to an embodiment, the control system 1720 may adjust the magnitude of a torque (or force) applied to the user. The control system 1720 may apply a force to at least one of the one or more wearable parts 1750 and 1755 worn by the user using the cables 1730 and 1735. The cables 1730 and 1735 may be elastic cables or inelastic cables. For example, the wearable parts 1750 and 1755 may be worn on the wrists of the user. Embodiments are not limited thereto, and the wearable parts 1750 and 1755 may be worn on the elbows of the user. The control system 1720 may adjust the magnitudes of the forces applied to the wearable parts 1750 and 1755 through the cables 1730 and 1735.

According to an embodiment, one ends of the cables 1730 and 1735 may be connected, directly or indirectly, to the gripping parts 1740 and 1745, respectively. The gripping parts 1740 and 1745 may be in a shape that the user may hold in the palms using fingers so that the user may easily adjust the lengths of the cables 1730 and 1735, and the shape of the gripping parts 1740 and 1745 is not limited to the described embodiments.

According to an embodiment, the wearable parts 1750 and 1755 may allow one ends of the cables 1730 and 1735 to be positioned closely to the body of the user even when the user does not hold the gripping parts 1740 and 1745.

According to an embodiment, the wearable parts 1750 and 1755 may each include an IMU.

In the example shown in FIG. 17A, the gripping parts 1740 and 1745 and the wearable parts 1750 and 1755 have separate shapes. However, depending on embodiments, the gripping part 1740 and the wearable part 1750 may be manufactured to have one shape as an integral body, and the gripping part 1745 and the wearable part 1755 may be manufactured to have one shape as an integral body.

An example of a frontal view of a user wearing the upper body-type wearable device is shown in FIG. 17B.

The wearable portion 1710 of the upper body-type wearable device 1700 may be worn on the upper body of the user.

When the wearable portion 1710 is worn on the upper body of the user, the control system 1720 may be placed on the back of the user. The gripping parts 1740 and 1745 and the wearable parts 1750 and 1755 of the upper body-type wearable device 1700 may be worn to be placed on the hands and wrists of the user. The lengths of the cables 1730 and 1735 may change according to motions of the arms of the user. For example, when the upper body-type wearable device 1700 operates in a daily mode or a game mode, the tension of the cables 1730 and 1735 may be controlled to such an extent that motions of the arms of the user may not be interfered with. For example, when the upper body-type wearable device 1700 operates in an exercise mode, the tension of the cables 1730 and 1735 may be controlled according to an exercise intensity to provide an external force to the user.

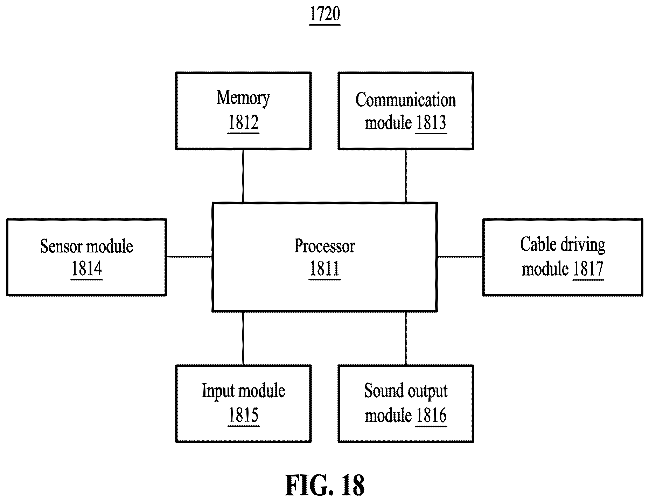

FIG. 18 is a block diagram illustrating an example of a configuration of a control system of an upper body-type wearable device according to an example embodiment.

Referring to FIG. 18, the upper body-type wearable device 1700 may be controlled by the control system 1720. The control system 1720 may include a processor 1811, a memory 1812, a communication module 1813, a sensor module 1814, an input module 1815, a sound output module 1816, and a cable drive module 1817. In an embodiment, at least one (e.g., the sound output module 1816) of these components may be omitted from the control system 1720, or one or more other components (e.g., a haptic module) may be added thereto. Each "module" herein may comprise circuitry.

According to an embodiment, the cable drive module 1817 may include a motor configured to generate a torque (or force) and a motor driver circuit configured to drive the motor. For example, the cable drive module 1817 may include a first motor configured to generate a tension or torque to be applied to the cable 1730 and a first motor driver circuit configured to drive the first motor, and include a second motor configured to generate a tension or torque to be applied to the cable 1735 and a second motor driver circuit configured to drive the second motor.

According to an embodiment, the sensor module 1814 may include a sensor circuit including at least one sensor. The sensor module 1814 may include one or more sensors configured to generate motion information of the user or motion information of the upper body-type wearable device 1700. For example, the sensor module 1814 may include an encoder configured to measure the lengths of the cables 1730 and 1735. For example, the sensor module 1814 may include a pulse sensor configured to measure the heart rate of the user. For example, the sensor module 1814 may further include at least one of a position sensor configured to obtain a position value of the upper body-type wearable device 1700, a proximity sensor configured to sense the proximity of an object, a biometric sensor configured to detect a biosignal of the user, or a temperature sensor configured to measure an ambient temperature.

According to an embodiment, the input module 1815 may receive a command or data to be used by a component (e.g., the processor 1811) of the upper body-type wearable device 1700 from the outside (e.g., the user) of the upper body-type wearable device 1700. The input module 1815 may include an input component circuit. The input module 1815 may include, for example, a key (e.g., a button) or a touch screen.

According to an embodiment, the sound output module 1816 may output a sound signal to the outside of the upper body-type wearable device 1700. The sound output module 1816 may provide auditory feedback to the user. For example, the sound output module 1816 may include a speaker configured to play back a guiding sound signal (e.g., an operation start sound, an operation error alarm, or an exercise start alarm), music content, or a guiding voice for auditorily informing predetermined information (e.g., exercise result information or exercise posture evaluation information).

According to an embodiment, the control system 1720 may further include a battery (not shown) configured to supply power to each component of the upper body-type wearable device 1700. The upper body-type wearable device 1700 may convert the power of the battery into power suitable for an operating voltage of each component of the upper body-type wearable device 1700 and supply the converted power to each component.

According to an embodiment, the cable drive module 1817 may generate tensions or torques to be applied to the cables 1730 and 1735 under the control of the processor 1811. The cable drive module 1817 may generate the tensions or torques to be applied to the cables 1730 and 1735 based on a control signal generated by the processor 1811.

According to an embodiment, the processor 1811 may control the overall operation of the upper body-type wearable device 1700 and generate a control signal for controlling the components (e.g., the communication module 1813 and the cable drive module 1817).

According to an embodiment, the processor 1811 may execute, for example, software to control at least one other component (e.g., a hardware or software component) of the upper body-type wearable device 1700 connected, directly or indirectly, to the processor 1811, and may perform a variety of data processing or computation. According to an embodiment, as at least part of data processing or computation, the processor 1811 may store instructions or data received from another component (e.g., the communication module 1813) in the memory 1812, process the instructions or data stored in the memory 1812, and store result data obtained as a result of processing in the memory 1812.

According to an embodiment, the processor 1811 may receive motion information (e.g., 3-axis acceleration and/or rotation angles) of the arms from the IMUs respectively positioned in the wearable parts 1750 and 1755.

According to an embodiment, the memory 1812 may store a variety of data used by the processor 1811. The data may include, for example, software, sensor data, and input data or output data for instructions related thereto. The memory 1812 may include a volatile memory or a non-volatile memory (e.g., a random-access memory (RAM), a dynamic RAM (DRAM), or a static RAM (SRAM)).

The communication module 1813, comprising communication circuitry, may support the establishment of a direct (e.g., wired) communication channel or a wireless communication channel between the processor 1811 and another component of the upper body-type wearable device 1700 or an external electronic device, and support the communication through the established communication channel. The communication module 1813 may include a communication circuit configured to perform a communication function. For example, the communication module 1813 may receive a control signal from an external device (e.g., at least one of the smart phone 120, the other wearable device 130, or the server 140) and transmit the sensor data obtained by the sensor module 1814 to the external device. According to an embodiment, the communication module 1813 may include a wireless communication module (e.g., a cellular communication module, a short-range wireless communication module, or a global navigation satellite system (GNSS) communication module), and/or a wired communication module. The communication module 1813 may include, for example, at least one of a Bluetooth communication circuit configured to perform Bluetooth communication, a wireless fidelity (WiFi) communication circuit configured to perform WiFi communication, or a cellular communication circuit configured to perform cellular communication (e.g., 4G communication, 5G communication, etc.).

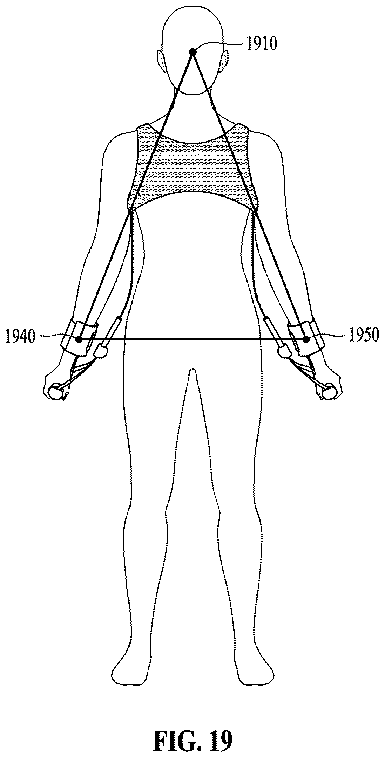

FIG. 19 is a diagram illustrating an example of determining a posture of a user by an upper body-type wearable device according to an example embodiment.

In the example shown in FIG. 19, according to an embodiment, the upper body-type wearable device 1700 (e.g., the processor 1811) may determine (or set) a first point (e.g., the first point 610 or the first point 710). For example, the processor 1811 may receive sensing data from a sensor capable of measuring distance (or position) from the other wearable device 130 (e.g., the wireless earphones 131 and/or the smart glasses 133) worn on the head of a user. The processor 1811 may calculate the coordinate value (or the position value) (e.g., the three-dimensional coordinate value) of the other wearable device 130 based on the sensing data received from the other wearable device 130, and set the point of the calculated coordinate value as a first point 1910. Embodiments are not limited thereto, the other wearable device 130 may calculate the coordinate value (or the position value) of the first point 1910 through the sensing data from the sensor capable of measuring distance (or position), and transmit the coordinate value (or the position value) of the first point 1910 to the upper body-type wearable device 1700. The processor 1811 may determine the point of the received coordinate value (or position value) to be the first point 1910.

According to an embodiment, the upper body-type wearable device 1700 (e.g., the processor 1811) may determine (or set) a fourth point 1940 corresponding to the right arm and a fifth point 1950 corresponding to the left arm.

For example, the processor 1811 may receive the acceleration information (e.g., the 3-axis acceleration) and/or rotation angle information (e.g., the roll, pitch, and yaw) of the right wrist from the IMU (or the IMU sensor) of the wearable part 1750, and calculate a coordinate value (or a position value) corresponding to the position of the right wrist (or the position of the IMU of the wearable part 1750) through the received acceleration information and/or rotation angle information. The processor 1811 may determine (or set) the point of the coordinate value corresponding to the position of the right wrist (or the position of the IMU of the wearable part 1750) to be the fourth point 1940. The processor 1811 may receive the acceleration information (e.g., the 3-axis acceleration) and/or rotation angle information (e.g., the roll, pitch, and yaw) of the left wrist from the IMU of the wearable part 1755, and calculate a coordinate value (or a position value) corresponding to the position of the left wrist (or the position of the IMU of the wearable part 1755) through the received acceleration information and/or rotation angle information. The processor 1811 may determine (or set) the point of the coordinate value corresponding to the position of the left wrist (or the position of the IMU of the wearable part 1755) to be the fifth point 1950.

Unlike the example shown in FIG. 19, the wearable parts 1750 and 1755 may be worn near the elbows of the user. In this case, the processor 1811 may set the point of the coordinate value corresponding to the position of the right elbow (or the position of the IMU of the wearable part 1750) as the fourth point, and set the point of the coordinate value corresponding to the position of the left elbow (or the position of the IMU of the wearable part 1755) as the fifth point.

According to an embodiment, the upper body-type wearable device 1700 (e.g., the processor 1811) may connect the first point 1910, the fourth point 1940, and the fifth point 1950 to each other to form a triangle. The processor 1811 may form the triangle by static modeling (e.g., setting and connecting the points 1910, 1940, and 1950).

According to an embodiment, in part (e.g., the control system 1720) of the upper body-type wearable device 1700, the origin (hereinafter, referred to as the "second origin") may be set (or determined) to calculate relative coordinate values of the points 1910, 1940, and 1950. The coordinate value of the second origin may be fixed. The processor 1811 may calculate the relative coordinate values of the points 1910, 1940, and 1950 based on the second origin.

According to an embodiment, the processor 1811 may determine the posture of the user based on the points 1910, 1940, and 1950. For example, the processor 1811 may determine that the head posture of the user is an abnormal posture (e.g., a turtle neck posture) in response to the determination that the first point 1910 is a predetermined level ahead of a line between the fourth point 1940 and the fifth point 1950. For example, the forward direction of the user may be the first axis (e.g., X-axis) direction. The processor 1811 may determine that the head posture of the user is the abnormal head posture (e.g., the turtle neck posture) when the difference between a first axis value (e.g., the X-axis value) of the relative coordinate value of the first point 1910 and a first axis value (e.g., the X-axis value) of the relative coordinate value of the fourth point 1940 is greater than or equal to a predetermined level in the front direction and/or when the difference between the first axis value (e.g., the X-axis value) of the relative coordinate value of the first point 1910 and a first axis value (e.g., the X-axis value) of the relative coordinate value of the fifth point 1950 is greater than or equal to the predetermined level in the front direction.

According to an embodiment, the processor 1811 of the upper body-type wearable device 1700 may determine that the user is performing an exercise (e.g., a walking exercise), through the motion information from the IMU. The processor 1811 may determine whether the head posture of the user who is performing a walking exercise is an abnormal head posture. The upper body-type wearable device 1700 may be wirelessly connected to the smart glasses 133, and the processor 1811 may control to display the head posture of the user and a recommended head posture on the smart glasses 133. When the head posture of the user is determined to be an abnormal head posture (e.g., a turtle neck posture), the processor 1811 may control a driving module (e.g., an actuator) of the upper body-type wearable device 1700 to output torques to pull the shoulders of the user back. The driving module may be a component that is physically distinguished from the cable drive module 1817 in the upper body-type wearable device 1700. When the driving module outputs torques to pull the shoulders of the user back, the user may perform a walking exercise with the shoulders pulled back. Accordingly, the abnormal head posture (e.g., the turtle neck posture) of the user may be corrected by the upper body-type wearable device 1700.

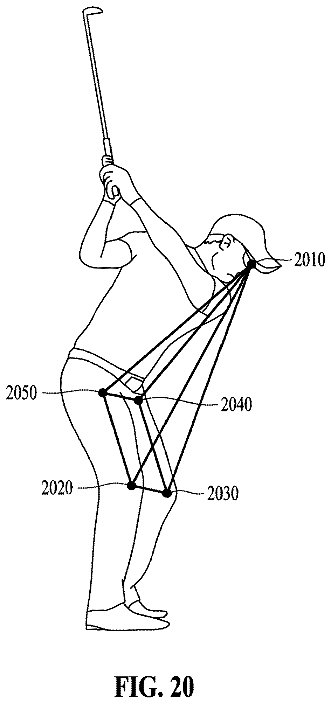

FIG. 20 is a diagram illustrating an example of an operation of a wearable device according to an example embodiment.

In the example shown in FIG. 20, a user may wear the wearable device 110 (e.g., the wearable device 300, the wearable device 300-1, or the wearable device 300-2), and wear the other wearable device 130 (e.g., the wireless earphones 131 and/or the smart glasses 133). The user may perform a golf exercise.

According to an embodiment, the processor 310 may calculate the coordinate value of a first point 2010 (e.g., the first point 1010) based on sensing data received from the other wearable device 130. The processor 310 may calculate the coordinate value of a second point 2020 (e.g., the second point 1020) corresponding to the right knee based on motion information received from the IMU 360-1. The processor 310 may calculate the coordinate value of a third point 2030 (e.g., the third point 1030) corresponding to the left knee based on motion information received from the IMU 360. The processor 310 may calculate the coordinate value of a sixth point 2050 corresponding to the right pelvis and the coordinate value of a seventh point 2040 corresponding to the left pelvis, considering the physical characteristics (e.g., the height) of the user.

According to an embodiment, the processor 310 may form a triangle (hereinafter, referred to as the "first triangle") through the first point 2010, the second point 2020, and the third point 2030, and form a triangle (hereinafter, referred to as the "second triangle") through the first points 2010, the sixth point 2050, and the seventh point 2040, and form a quadrangle (hereinafter, referred to as the "pelvic-knee quadrangle") through the second point 2020, the third point 2030, the sixth point 2050, and the seventh point 2040.

According to an embodiment, the processor 310 may determine the golf swing posture of the user using the first triangle, the second triangle, and the pelvis-knee quadrangle. For example, the processor 310 may determine whether the golf swing posture of the user is a correct swing posture through changes in the shapes of the first triangle, the second triangle, and the pelvis-knee quadrangle. When the golf swing posture of the user is determined to be an incorrect swing posture, the processor 310 may control to provide a guide for a correct swing posture. For example, the processor 310 may display the golf swing posture of the user and the correct swing posture on the smart glasses 133, and display a guide to correct the golf swing posture of the user to the correct swing posture on the smart glasses 133. As another example, the processor 310 may output a voice guide to correct the golf swing posture of the user to the correct swing posture through a speaker of the wearable device 110 or the wireless earphones 131.

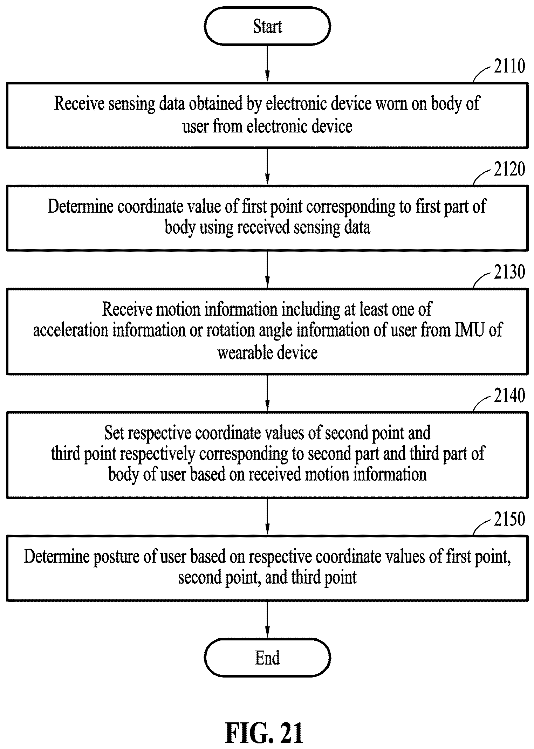

FIG. 21 is a flowchart illustrating an example of an operating method of a wearable device according to an embodiment.

Referring to FIG. 21, in operation 2110, according to an embodiment, the wearable device 110 (e.g., the wearable device 300, the wearable device 300-1, or the wearable device 300-2) may receive sensing data obtained by an electronic device (e.g., the other wearable device 130) worn on the body of a user from the electronic device.

In operation 2120, the wearable device 110 may determine a coordinate value (or relative coordinate value) of a first point (e.g., the first point 1010) corresponding to a first part (e.g., the head) of the body using the received sensing data.

In operation 2130, the wearable device 110 may receive motion information including at least one of acceleration information or rotation angle information of the user from an IMU (or an IMU sensor) (e.g., the IMU 360 and/or the IMU 360-1) of the wearable device 110.

In operation 2140, the wearable device 110 may determine respective coordinate values (or relative coordinate values) of a second point (e.g., the second point 1020) and a third point (e.g., the third point 1030) respectively corresponding to a second part (e.g., the right knee or the right hip joint) and a third part (e.g., the left knee or the left hip joint) of the body of the user based on the received motion information.

In operation 2150, the wearable device 110 may determine a posture of the user based on the respective coordinate values (or relative coordinate values) of the first point 1010, the second point 1020, and the third point 1030.

According to an embodiment, in operation 2150, the processor 310 may determine whether a polygon (e.g., a quadrangle) with the second point 1020 and the third point 1030 as diagonal points is formed based on the coordinate value of the second point 1020 and the coordinate value of the third point 1030. The processor 310 may determine that the user is walking in response to the determination that the polygon is formed. The processor 310 may determine that the user is not walking in response to the determination that the polygon is not formed.

According to an embodiment, in operation 2150, the processor 310 may determine whether a projection point of the coordinate value (or relative coordinate value) of the first point 1010 is positioned in an area (e.g., the area 1110 of FIG. 11) formed based on the second point 1020 and the third point 1030. The processor 310 may determine that the user is looking at the front in response to the determination that the projection point of the coordinate value (or relative coordinate value) of the first point is positioned in the area 1110. The processor 310 may determine that the user is not looking at the front in response to the determination that the projection point of the coordinate value (or relative coordinate value) of the first point is positioned out of the area 1110.

According to an embodiment, in operation 2150, the processor 310 may calculate a first distance between the second point 1020 and the third point 1030 through the coordinate value (or relative coordinate value) of the second point 1020 and the coordinate value (or relative coordinate value) of the third point 1030. The processor 310 may calculate a second distance between one of the second point 1020 and the third point 1030 and the projection point of the first point 1010. The processor 310 may determine a head posture of the user to be a normal posture when a difference between n times (e.g., "0.5" times) the calculated first distance and the calculated second distance is within a predetermined range. The processor 310 may determine the head posture to be an abnormal posture when the difference between n times (e.g., "0.5" times) the calculated first distance and the calculated second distance is beyond the predetermined range.

According to an embodiment, in operation 2150, the processor 310 may determine a vector of the second point 1020 through a coordinate value of a reference point (e.g., the reference point 910) of the second point 1020 and the coordinate value of the second point 1020. The processor 310 may determine a vector of the third point 1030 through a coordinate value of a reference point (e.g., the reference point 911) of the third point 1030 and the coordinate value of the third point 1030. The processor 310 may determine whether a gait posture of the user is an abnormal gait posture based on at least one of the vector of the second point 1020 or the vector of the third point 1030.

For example, the processor 310 may calculate an angle between a first reference line pointing in a front direction of the user from the reference point 910 of the second point 1020 and the vector of the second point 1020. The processor 310 may calculate an angle between a second reference line pointing in the front direction of the user from the reference point 911 of the third point 1030 and the vector of the third point. The processor 310 may determine the gait posture of the user to be a first abnormal gait posture (e.g., an out-toeing posture) in response to the determination that at least one of the calculated angles exceeds a predetermined range (e.g., "0" to "15" degrees) toward the outside of the front direction. The processor 310 may determine the gait posture of the user to be a second abnormal gait posture (e.g., an in-toeing posture) in response to the determination that at least one of the calculated angles exceeds the predetermined range (e.g., "0" to "15" degrees) toward the inside of the front direction.

According to an embodiment, the processor 310 may transmit information about the head posture (e.g., whether the head posture of the user is an abnormal posture) to the electronic device so as for a screen (e.g., the screen 1303 of FIG. 13) visualizing the head posture of the user to be displayed on the electronic device.

According to an embodiment, the processor 310 may determine a first timepoint at which a first foot of the user contacts a ground through the IMU (e.g., the IMU 360 and/or the IMU 360-1), and determine a second timepoint at which a second foot of the user contacts the ground through the IMU (e.g., the IMU 360 and/or the IMU 360-1). The processor 310 may calculate a step length of the user using a difference between the determined second timepoint and the determined first timepoint. The processor 310 may transmit the calculated step length, information about a recommended gait (e.g., a direction of the recommended gait), and information about a gait of the user (e.g., a gait angle) to the electronic device such that a screen (e.g., the screen 1301 of FIG. 13) visualizing the calculated step length, the recommended gait for the user, and the gait of the user may be displayed on the electronic device.

According to an embodiment, the processor 310 may form a polygon with the second point and the third point as diagonal points, and determine a walking state of the user based on the polygon being formed. For example, the processor 310 may determine the walking state of the user to be a third state or a fourth state by checking in real time the features (e.g., the shape, etc.) of the polygon being formed. The shape of the polygon when the walking state is the third state may be different from the shape of the polygon when the walking state is the fourth state. The processor 310 may determine the walking state of the user to be the third state or the fourth state through the shape of the polygon being formed. When a polygon with the second point and the third point as diagonal points is not formed, the processor 310 may determine the walking state of the user to be, for example, a first state or a second state.

According to an embodiment, the processor 310 may form a triangle based on the first point, the second point, and the third point, and determine the posture (e.g., the gait posture, the walking posture, the head posture, etc.) of the user using the formed triangle and the formed polygon (e.g., the polygon with the second point and the third point as diagonal points). The processor 310 may analyze the posture (e.g., the gait posture, the walking posture, the head posture, etc.) of the user in real time by analyzing at least one of the shape of the formed triangle or the shape of the formed polygon.

According to an embodiment, when one of the first foot and the second foot of the user is a rear foot contacting the ground and the other one is a front foot in the air, the processor 310 may predict a gait in a front direction of one of the second point 1020 and the third point 1030, corresponding to the front foot, and determine the predicted gait to be the recommended gait for the user.

According to an embodiment, the wearable device 110 may include a vibration module, comprising a motor (e.g., a vibration motor) and/or circuitry, to output vibration. The processor 310 may control the vibration module to output vibration in such a pattern as to move from the outside to the inside when the gait posture of the user is determined to be the first abnormal gait posture. The processor 310 may control the vibration module to output vibration in such a pattern as to move from the inside to the outside when the gait posture of the user is determined to be the second abnormal gait posture.

According to an embodiment, the processor 310 may determine a rotation angle value indicating how much the driving module 30 is to be inclined based on at least one of the calculated angles (e.g., the angle between the first reference line and the vector of the second point 1020 and the angle between the second reference line and the vector of the third point 1030), respective directions of the second point (e.g., the second point 620 or the second point 720) and the third point (e.g., the third point 630 or the third point 730) when the user is standing, or a matching rate calculated based on the number of matches of steps of the user with recommended steps. The processor 310 may control the driving module 30 to be inclined (or rotated) inward by the determined rotation angle value when the gait posture of the user is determined to be the first abnormal gait posture. The processor 310 may control the driving module 30 to be inclined (or rotated) outward by the determined rotation angle value when the gait posture of the user is determined to be the second abnormal gait posture.

According to an embodiment, the processor 310 may control the wearable device 110 or the electronic device (e.g., the other wearable device 130) to provide a guide that helps the gait of the user match the recommended gait.

According to an embodiment, the processor 310 may perform static modeling on the standing user. The processor 310 may receive second sensing data obtained by the electronic device (e.g., the other wearable device 130) from the electronic device when the user is standing. The processor 310 may calculate a first coordinate value based on the received second sensing data and determine a point having the calculated first coordinate value to be the first point (e.g., the first point 610, the first point 710, or the first point 1010). The processor 310 may receive at least one of second acceleration information or second rotation angle information of the user from the IMU (e.g., the IMU 360 and/or the IMU 360-1) when the user is standing. The processor 310 may calculate a second coordinate value and a third coordinate value based on the received at least one of the second acceleration information or the second rotation angle information. The processor 310 may determine a point having the calculated second coordinate value to be the second point (e.g., the second point 620, the second point 720, or the second point 1020). The processor 310 may determine a point having the calculated third coordinate value to be the third point (e.g., the third point 630, the third point 730, or the third point 1030).

According to an embodiment, the processor 310 may determine the head posture of the user to be a normal head posture when a difference between a first axis value of one of the second coordinate value and the third coordinate value and a first axis value of the first coordinate value is within a predetermined level. The processor 310 may determine the head posture of the user to be an abnormal head posture when the difference between the first axis value of one of the second coordinate value and the third coordinate value and the first axis value of the first coordinate value exceeds the predetermined level.

Each embodiment herein may be used in combination with any other embodiment(s) described herein.

According to an embodiment, the processor 310 may determine a direction of the second point (e.g., the second point 920 or the second point 930) when the user is standing based on the coordinate value of the reference point 910 of the second point 920 or 930 and the second coordinate value. The processor 310 may determine a direction of the third point (e.g., the third point 921 or the third point 931) when the user is standing based on the coordinate value of the reference point 911 of the third point 921 or 931 and the third coordinate value. The processor 310 may determine a front direction when the user is standing, through the direction of the second point and the direction of the third point. The processor 310 may calibrate the coordinate value of the reference point 910 of the second point and the coordinate value of the reference point of the third point 911 such that the determined front direction is a reference direction used to determine a gait posture of the user.

According to an embodiment, the wearable device 110 (e.g., the wearable device 300-2 of FIG. 3C) may include the plurality of IMUs 360 and 360-1. The first IMU (e.g., the IMU 360-1) may obtain first motion information including at least one of acceleration information or rotation angle information of a second part (e.g., the right knee or the right hip joint) of the body of the user. The second IMU (e.g., the IMU 360) may obtain second motion information including at least one of acceleration information or rotation angle information of a third part (e.g., the left knee or the left hip joint) of the body of the user. The processor 310 may receive sensing data obtained by the electronic device 130 worn on the first part (e.g., the head) of the body of the user from the electronic device 130, and determine a coordinate value (or relative coordinate value) of a first point 1010 corresponding to the first part of the body using the received sensing data. The processor 310 may receive the first motion information and the second motion information respectively from the first IMU and the second IMU. The processor 310 may determine a coordinate value (or relative coordinate value) of a second point 1020 corresponding to the second part of the body based on the received first motion information. The processor 310 may determine a coordinate value (or relative coordinate value) of a third point 1030 corresponding to the third part of the body based on the received second motion information. The processor 310 may determine a posture of the user based on the respective coordinate values (or relative coordinate values) of the first point 1010, the second point 1020, and the third point 1030.

The embodiments described with reference to FIGS. 1 to 20 may apply to the operating method of the wearable device 110 of FIG. 21.

The units described herein may be implemented using a hardware component, a software component and/or a combination thereof. A processing device may be implemented using one or more general-purpose or special-purpose computers, such as, for example, a processor, a controller and an arithmetic logic unit (ALU), a digital signal processor (DSP), a microcomputer, a field-programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter.

The above-described devices may be configured to act as one or more software modules in order to perform the operations of the above-described examples, or vice versa.

A number of embodiments have been described above. Nevertheless, it should be understood that various modifications may be made to these embodiments. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and/or equivalents of the claims are within the scope of the following claims.

While the disclosure has been illustrated and described with reference to various embodiments, it will be understood that the various embodiments are intended to be illustrative, not limiting. It will further be understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

## Claims

1. A wearable device (110) comprising:
a communication module (390), comprising communication circuitry, configured to communicate with an electronic device to be worn on a body of a user;
a driving module (130), comprising a motor and/or circuitry, configured to generate a torque and provide the user with the generated torque;
at least one inertial measurement unit (IMU) sensor (360) configured to obtain motion information comprising at least one of acceleration information or rotation angle information of the user; and
at least one processor (310), comprising processing circuitry, individually and/or collectively configured to: control the driving module to provide the torque to the user, receive sensing data obtained by the electronic device from the electronic device through at least the communication module, determine a coordinate value of a first point corresponding to a first part of the body using the received sensing data, receive the obtained motion information from the at least one IMU sensor, determine respective coordinate values of a second point and a third point respectively corresponding to a second part and a third part of the body based on the received motion information, and determine a posture of the user based on the respective coordinate values of the first point, the second point, and the third point.

2. The wearable device of claim 1, wherein at least one processor is individually and/or collectively configured to determine whether a projection point of the coordinate value of the first point is positioned in an area formed based on the second point and the third point, determine that the user is looking at the front in response to the determination that the projection point is positioned in the area, and determine that the user is not looking at the front in response to the determination that the projection point is positioned out of the area.

3. The wearable device of any one of claims 1 and 2, wherein at least one processor is individually and/or collectively configured to calculate a first distance between the second point and the third point through the coordinate value of the second point and the coordinate value of the third point, calculate a second distance between one of the second point and the third point and the projection point of the first point, determine a head posture of the user to be a normal posture when a difference between n times the calculated first distance and the calculated second distance is within a predetermined range, and determine the head posture to be an abnormal posture when the difference is beyond the predetermined range, and
n comprises "0.5".

4. The wearable device of claim 3, wherein at least one processor is individually and/or collectively configured to control to transmit information about the head posture to the electronic device so as for a screen visualizing the head posture of the user to be displayed on the electronic device.

5. The wearable device of any one of claims 1 to 4, wherein at least one processor is individually and/or collectively configured to determine a vector of the second point through a coordinate value of a reference point of the second point and the coordinate value of the second point, determine a vector of the third point through a coordinate value of a reference point of the third point and the coordinate value of the third point, and determine whether a gait posture of the user is an abnormal gait posture based on at least one of the vector of the second point or the vector of the third point.

6. The wearable device of claim 5, wherein at least one processor is individually and/or collectively configured to calculate an angle between a first reference line pointing in a front direction of the user from the reference point of the second point and the vector of the second point, calculate an angle between a second reference line pointing in the front direction of the user from the reference point of the third point and the vector of the third point, determine the gait posture to be a first abnormal gait posture in response to the determination that at least one of the calculated angles exceeds a predetermined range toward the outside of the front direction, and determine the gait posture to be a second abnormal gait posture in response to the determination that at least one of the calculated angles exceeds the predetermined range toward the inside of the front direction.

7. The wearable device of claim 6, further comprising:
a vibration module, comprising a motor and/or circuitry, configured to output vibration,
wherein at least one processor is individually and/or collectively is configured to control the vibration module to output vibration in such a pattern as to move from the outside to the inside when the gait posture of the user is determined to be the first abnormal gait posture, and control the vibration module to output vibration in such a pattern as to move from the inside to the outside when the gait posture of the user is determined to be the second abnormal gait posture; or,
wherein at least one processor is individually and/or collectively configured to determine a rotation angle value indicating how much the driving module is to be inclined based on at least one of the calculated angles, respective directions of the second point and the third point when the user is standing, or a matching rate calculated based on the number of matches of steps of the user with recommended steps, control the driving module to be inclined inward by the determined rotation angle value when the gait posture of the user is determined to be the first abnormal gait posture, and control the driving module to be inclined outward by the determined rotation angle value when the gait posture of the user is determined to be the second abnormal gait posture.

8. The wearable device of any one of claims 1 to 7, wherein at least one processor is individually and/or collectively configured to determine whether a polygon with the second point and the third point as diagonal points is formed based on the coordinate value of the second point and the coordinate value of the third point, determine that the user is walking in response to the determination that the polygon is formed, and determine that the user is not walking in response to the determination that the polygon is not formed.

9. The wearable device of any one of claims 1 to 8, wherein at least one processor is individually and/or collectively configured to determine a first timepoint at which a first foot of the user contacts a ground through the at least one IMU sensor, determine a second timepoint at which a second foot of the user contacts the ground through the at least one IMU sensor, calculate a step length of the user using a difference between the determined second timepoint and the determined first timepoint, and transmit the calculated step length, information about a recommended gait for the user, and information about a gait of the user to the electronic device such that a screen visualizing the calculated step length, the recommended gait, and the gait of the user is displayed on the electronic device.

10. The wearable device of claim 9, wherein at least one processor is individually and/or collectively configured to, when one of the first foot and the second foot is a rear foot contacting the ground and the other one is a front foot in the air, predict a gait in a front direction of one of the second point and the third point, corresponding to the front foot, and determine the predicted gait to be the recommended gait; or,
wherein at least one processor is individually and/or collectively configured to control to provide a guide that helps the gait of the user match the recommended gait.

11. The wearable device of any one of claims 1 to 10, wherein at least one processor is individually and/or collectively configured to receive second sensing data obtained by the electronic device from the electronic device when the user is standing, calculate a first coordinate value based on the received second sensing data, determine a point having the calculated first coordinate value to be the first point, receive at least one of second acceleration information or second rotation angle information of the user from the at least one IMU sensor when the user is standing, calculate a second coordinate value and a third coordinate value based on the received at least one of the second coordinate value or the second rotation angle information, determine a point having the calculated second coordinate value to be the second point, and determine a point having the calculated third coordinate value to be the third point.

12. The wearable device of claim 11, wherein at least one processor is individually and/or collectively configured to determine the head posture of the user to be a normal head posture when a difference between a first axis value of one of the second coordinate value and the third coordinate value and a first axis value of the first coordinate value is within a predetermined level, and determine the head posture of the user to be an abnormal head posture when the difference exceeds the predetermined level; or,
wherein at least one processor is individually and/or collectively configured to determine a direction of the second point when the user is standing based on the coordinate value of the reference point of the second point and the second coordinate value, determine a direction of the third point when the user is standing based on the coordinate value of the reference point of the third point and the third coordinate value, determine a front direction when the user is standing, through the direction of the second point and the direction of the third point, and calibrate the coordinate value of the reference point of the second point and the coordinate value of the reference point of the third point such that the determined front direction is a reference direction used to determine a gait posture of the user.

13. The wearable device of any one of claims 1 to 12, wherein at least one processor is individually and/or collectively configured to form a polygon with the second point and the third point as diagonal points, and determine a walking state of the user based on the polygon being formed.

14. The wearable device of claim 13, wherein at least one processor is individually and/or collectively configured to form a triangle based on the first point, the second point, and the third point, and determine the posture using the formed triangle and the formed polygon.

15. An operating method of a wearable device (110), the operating method comprising:
receiving sensing data obtained by an electronic device (130) worn on a body of a user from the electronic device;
determining a coordinate value of a first point corresponding to a first part of the body using the received sensing data;
receiving motion information comprising at least one of acceleration information or rotation angle information of the user from an inertial measurement unit (IMU) sensor (360) of the wearable device;
determining respective coordinate values of a second point and a third point respectively corresponding to a second part and a third part of the body based on the received motion information; and
determining a posture of the user based on the respective coordinate values of the first point, the second point, and the third point.
